(19) Europäisches Patentamt
European Patent Office
Office européen des brevets

(11) Publication number: **0 000 644**
**A2**

(12) **EUROPEAN PATENT APPLICATION**

(21) Application number: 78300168.8

(22) Date of filing: 19.07.78

(51) Int. Cl.$^2$: C 07 C 65/20, C 07 C 101/18, C 07 C 143/74, C 07 C 143/822, C 07 C 91/16, C 07 C 101/04, A 61 K 31/00, C 07 C 69/76, C 07 C 65/14, C 07 C 31/34, C 07 C 43/12//C 07 C 63/60, C 07 69/76, C 07 C 59/22, C 07 D 317/30, C 07 D 317/18

(31) Priority: 19.07.77 GB 30229/77

(43) Date of publication of application: 07.02.79 Bulletin 79/3

(84) Designated contracting states: BE CH DE FR GB LU NL SE

(71) Applicant: Fujisawa Pharmaceutical Co. Ltd.
3,4-chome Doshomachi Higashi-ku
Osaka. (JP)

(72) Inventor: Kamiya, Takashi
No. 1-11-16, Furuedai
Suita. (JP)

(72) Inventor: Shiokawa, Youichi
No. 1319, Makito-cho
Takatsuki. (JP)

(74) Representative: Venner, Harold Arthur
Lake Rugby Chambers 2, Rugby Street
London, WC1N 3QU.. (GB)

(54) **Lower cycloalkyl substituted-benzene derivatives, the preparation thereof and their pharmaceutical compositions.**

(57) Lower cycloalkyl substituted-benzene derivatives, the preparation thereof and their pharmaceutical compositions.

Lower cycloalkyl substituted-benzene derivatives of the formula:–

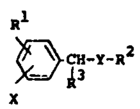

wherein $R^1$ is lower cycloalkyl,

$R^2$ is carboxy, esterified carboxy, hydroxymethyl, lower alkoxymethyl or acyloxymethyl,

$R^3$ is lower alkanoyl or lower alkyl substituted with hydroxy, amino, lower alkylamino, di(lower)alkylamino, acylamino, lower alkoxy, acyl or acyloxy, in which the lower alkyl moieties of the di(lower)alkyl amino radical may be joined together to form a heterocyclic ring containing the nitrogen atom and the carbonyl function of the acyl radical, which is a substituent on the lower alkyl radical, may be protected,

X is hydrogen or halogen, and

Y is a valency bond or lower alkylene;

and pharmaceutically acceptable salts thereof; pharmaceutical composition comprising the same; and methods for treatment of inflammation by administering the same to mammals.

EP 0 000 644 A2

The present invention is concerned with new lower cycloalkyl substituted-benzene derivatives. More particularly, the present invention is concerned with new lower cycloalkyl substituted-benzene derivatives and with the pharmaceutically-acceptable salts thereof, which have anti-inflammatory activity, with the processes for the preparation thereof, with pharmaceutical compositions comprising them and with methods for the treatment of inflammation by administering the new derivatives to mammals.

The new lower cycloalkyl substituted-benzene derivatives of this invention are compounds of the general formula:-

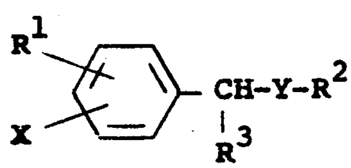

(I)

wherein $R^1$ is a lower cycloalkyl radical,

$R^2$ is a carboxy, esterified carboxy, hydroxymethyl, lower alkoxymethyl or acyloxymethyl radical,

$R^3$ is a lower alkanoyl radical or a lower alkyl radical substituted with

a hydroxy, amino, lower alkylamino, di(lower)alkylamino, acylamino, lower alkoxy, acyl or acyloxy radical, in which the lower alkyl moieties of the di(lower)alkyl amino radical may be joined together to form a heterocyclic ring containing the nitrogen atom and the carbonyl function of the acyl radical, which is a substituent on the lower alkyl radical, may be protected,

X is a hydrogen or halogen atom, and

Y is a valency bond or a lower alkylene radical;

and the pharmaceutically acceptable salts thereof.

In this specification, it is to be understood that the term "lower" used in connection with an alkyl, alkenyl, alkoxy, alkylene and alkanoyl radical is intended to mean the radicals containing up to eight carbon atoms, unless otherwise indicated.

As to the radicals as defined in the above, the more detailed explanation will be made and preferred examples thereof will be illustrated in the following.

The lower cycloalkyl radical for $R^1$ can contain 3 to 8 carbon atoms, preferably 5 to 7, examples of which may include cyclopropyl, cyclobutyl, cyclopentyl, cyclohexyl, cycloheptyl, cyclooctyl and the like.

The esterified carboxy radical for $R^2$ may include substituted and unsubstituted lower alkoxy carbonyl, for example, methoxycarbonyl, ethoxycarbonyl, n-propoxycarbonyl, isopropoxycarbonyl, n-butoxycarbonyl, isobutoxycarbonyl, tert.-butoxycarbonyl, pentyloxycarbonyl, hexyloxycarbonyl, chloromethoxycarbonyl, bromoethoxycarbonyl, trifluoromethoxycarbonyl, 2,2,2-trichloroethoxycarbonyl, 2,2,2-tribromoethoxycarbonyl, 1-cyclopropylethoxycarbonyl, cyanomethoxycarbonyl, cyanoethoxycarbonyl, dimethylaminoethoxycarbonyl,

dimethylaminopropoxycarbonyl, phenoxymethoxycarbonyl, phenoxyethoxycarbonyl, phenoxypropoxycarbonyl, phenylthiomethoxycarbonyl, phenylthioethoxycarbonyl, phenylthiopropoxycarbonyl, benzenesulphinylmethoxycarbonyl, benzenesulphinylethoxycarbonyl, benzoylmethoxycarbonyl, toluoylethoxycarbonyl, 3,4,5-trimethoxyphenylpropoxycarbonyl, pyridylmethoxycarbonyl, furfuryloxycarbonyl, thenyloxycarbonyl, 1,4-dioxanylmethoxycarbonyl, pyrrolidinylethoxycarbonyl, 4-methylpiperidinylethoxycarbonyl and the like; lower alkenyloxycarbonyl, for example, vinyloxycarbonyl, 1-propenyloxycarbonyl, allyloxycarbonyl, 3-butenyloxycarbonyl and the like; lower alkynyloxycarbonyl, for example, ethynyloxycarbonyl, propargyloxycarbonyl, 3-butynyloxycarbonyl, 4-pentynyloxycarbonyl and the like; lower cycloalkoxycarbonyl, for example, cyclopentyloxycarbonyl, cyclohexyloxycarbonyl and cycloheptyloxycarbonyl; substituted and unsubstituted aryloxycarbonyl, for example, phenoxycarbonyl tolyloxycarbonyl, xylyloxycarbonyl, naphthoxycarbonyl, 4-chlorophenoxycarbonyl, 3,5-dibromophenoxycarbonyl, pentachlorophenoxycarbonyl, 4-methoxyphenoxycarbonyl and the like; substituted and unsubstituted ar(lower)alkoxycarbonyl, for example, benzyloxycarbonyl, phenethyloxycarbonyl, 3,4-dichlorobenzyloxycarbonyl, 4-nitrobenzyloxycarbonyl, 4-methoxybenzyloxycarbonyl, 3,4,5-trimethoxybenzyloxycarbonyl, 3,4,5-trimethoxyphenylpropoxycarbonyl, 4-hydroxy-3,5-di-(tert.-butyl)-benzyloxycarbonyl and the like; ar(lower)alkenyloxycarbonyl, for example, cinnamyloxycarbonyl and the like; heterocyclic-oxycarbonyl, for example, pyridyloxycarbonyl, picolyloxycarbonyl, tetrahydropyranyloxycarbonyl, tetrahydrofuryloxycarbonyl, quinolyloxycarbonyl, pyrazolyloxycarbonyl and the like; tri-(lower)alkylsilyloxycarbonyl, for example, trimethylsilyloxycarbonyl, triethylsilyloxycarbonyl and the like; N,N-disubstituted aminoxycarbonyl,

for example, isopropylideneaminooxycarbonyl, cyanobenzylideneaminooxycarbonyl, phthalimidooxycarbonyl, succinimidooxycarbonyl, 1-benzotriazolyloxycarbonyl and the like.

The lower alkoxymethyl radicals for $R^2$ may include a methyl radical substituted by a lower alkoxy radical, for example, a methoxy, ethoxy, n-propoxy, isopropoxy, n-butoxy, isobutoxy, tert.-butoxy, pentyloxy, hexyloxy, heptyloxy and the like.

The acyl moiety of the acyloxymethyl radical for $R^2$ may include substituted and unsubstituted lower alkanoyl, such as formyl, lower alkanoyl (e.g. acetyl, propionyl, butyryl, pentanoyl, hexanoyl, heptanoyl, trifluoroacetyl, chloroacetyl, etc.) or higher alkanoyl (e.g. nonanoyl, decanoyl, palmitoyl, etc.); lower alkoxycarbonyl, such as methoxycarbonyl, ethoxycarbonyl, n-propoxycarbonyl, isopropoxycarbonyl, n-butoxycarbonyl, isobutoxycarbonyl, tert.-butoxycarbonyl, pentyloxycarbonyl, hexyloxycarbonyl and the like; substituted and unsubstituted aroyl such as benzoyl, toluoyl, xyloyl, naphthoyl, chlorobenzoyl, nitrobenzoyl, hydroxybenzoyl or the like; substituted and unsubstituted ar(lower)-alkanoyl such as phenylacetyl, phenylpropionyl, tolylacetyl, naphthylacetyl, chlorophenylacetyl, methoxyphenylacetyl, hydroxyphenylacetyl or the like; substituted and unsubstituted aryloxy(lower)alkanoyl such as phenoxyacetyl, nitrophenoxyacetyl or the like;

heterocyclic carbonyl such as furoyl, thenoyl, nicotinoyl and isonicotinoyl; lower alkanesulphonyl such as mesyl, ethanesulphonyl, propanesulphonyl, butanesulphonyl, pentanesulphonyl or the like; and arenesulphonyl such as benzenesulphonyl, toluenesulphonyl, naphthalenesulphonyl or the like.

The lower alkanoyl radical for $R^3$ may include the lower alkanoyl radical as illustrated above for the acyl moiety of the acyloxymethyl radical for $R^2$, and

preferably, formyl, acetyl and propionyl.

The lower alkyl moiety of the lower alkylamino and di(lower)alkylamino radicals which are substituents on the lower alkyl radical for $R^3$ may include normal and branched chain alkyl such as methyl, ethyl, n-propyl, isopropyl, n-butyl, isobutyl, sec.-butyl, tert.-butyl, pentyl, neopentyl, hexyl or the like.

The acyl radical and the acyl moiety of the acylamino and acyloxy radicals which are substituents on the lower alkyl radical for $R^3$ may be the same as those illustrated hereinbefore as examples of the acyl moiety of the acyloxymethyl radical for $R^2$.

The lower alkoxy radical substituted on the lower alkyl radical for $R^3$ may be the same as those illustrated hereinbefore as examples of the lower alkoxy moiety of the lower alkoxymethyl radical for $R^2$.

The heterocyclic ring containing the nitrogen atom which is formed by joining the lower alkyl moieties of the di(lower)alkylamino(lower)alkyl radical for $R^3$ may include N-containing 3 to 7 membered heterocyclic group, such as 1-aziridinyl, 1-azetidinyl, 1-pyrrolidinyl or piperidino.

The protective group for the carbonyl function of the acyl(lower)alkyl radical for $R^3$ preferably includes the acetal type of protective group, preferred examples of the protected carbonyl moiety including 1,3-dioxolan-2-ylidene, 4-methyl-1,3-dioxolan-2-ylidene, dimethoxymethylene, diethoxymethylene and the like.

The halogen atom X can be fluorine, chlorine, bromine and iodine.

The lower alkylene radical Y includes branched and straight-chained radicals, such as methylene, ethylene, propylene, trimethylene, tetramethylene, pentamethylene, hexamethylene, 2,2-dimethyltrimethylene and the like.

Pharmaceutically acceptable salts of the compounds (I) include conventional non-toxic salts, such as inorganic base salts, for example, metal salts, such as alkali metal salts (e.g. sodium salts, potassium salts, etc.), and alkaline earth metal salts (e.g. calcium salts, magnesium salts, etc.), and ammonium salts; organic base salts, for example, amine salts (e.g. trimethylamine, triethylamine, ethanolamine, diethanolamine, pyridine and dicyclohexylamine salts, etc.); inorganic acid salts (e.g. hydrochlorides, hydrobromides, sulphates, etc.); and organic acid salts (e.g. acetates, maleates, tartrates, lactates, methanesulphonates, etc.).

With regard to the definition of the compounds of general formula (I) as defined and explained above, it is to be noted that the definition "a lower alkyl radical substituted with a hydroxy, amino, lower alkylamino, di(lower)alkylamino, acylamino, lower alkoxy, acyl or acyloxy radical" for $R^3$ can also be expressed by the wording "a hydroxy(lower)alkyl, amino(lower)alkyl, lower alkylamino(lower)alkyl, di(lower)alkylamino(lower)-alkyl, acylamino(lower)alkyl, lower alkoxy(lower)alkyl, acyl(lower)alkyl or acyloxy(lower)alkyl". It is also to be noted that, when $R^3$ of the compounds (I) is an acyl radical, the compounds (I) include the tautomeric isomers thereof, as will be apparent from the following description.

The compounds (I) of the present invention can be prepared by the methods illustrated in the following:

(1) <u>Preparation A</u>:

A compound of the general formula (Ia):-

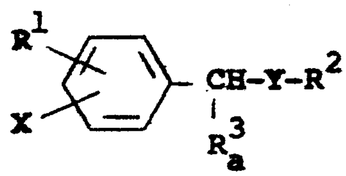

(Ia)

wherein $R^1$, $R^2$, X and Y have the same meanings as above, and $R_a^3$ is a lower alkanoyl radical, can be prepared by reacting a compound of the general formula:

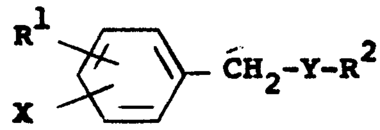

(II)

wherein $R^1$, $R^2$, X and Y have the same meanings as above, with a lower alkanoic acid of the general formula:

$$R_a^3 - OH \qquad (III)$$

wherein $R_a^3$ has the same meaning as above, or with a reactive derivative thereof, preferably in the presence of a strong base.

Of the starting compounds (II), methyl 4-cyclohexylphenyl acetate (i.e. the compound (II) wherein $R^1$ is cyclohexyl, X is hydrogen atom, Y is bond and $R^2$ is methoxycarbonyl) is known and can be prepared, for example, by the method described in Chemical Abstracts 66, 10765b/1967 and the other starting compounds (II) can be prepared in substantially the same manner as described therein.

Preferred examples of the reactive derivatives of the lower alkanoic acids (II) include acid halides, such as acid chlorides and acid bromides; acid azides; acid anhydrides, i.e. mixed and symmetrical acid anhydrides, and activated esters, including those illustrated hereinbefore as the esterified carboxy radical for $R^2$.

Preferred examples of the strong bases to be used in this process include alkali metal hydrides, such as sodium or potassium hydride; alkali metal amides, such as sodium or potassium hydride; alkali metal amides, such as lithamide, sodamide and potassamide; alkali metal alkoxides, such as sodium methoxide, sodium ethoxide, sodium propoxide, potassium methoxide and

potassium ethoxide; alkali metal phenoxides, such as lithium phenoxide, sodium phenoxide and potassium phenoxide; organic lithium compounds, such as phenyl lithium, butyl lithium, diethylaminolithium and N-methylanilinolithium and the like.

The reaction is usually carried out in a conventional organic solvent, such as diethyl ether, tetrahydrofuran, benzene, diethyl carbonate, N,N'-dimethylformamide, pyridine or the like.

When the lower alkanoic acid (III) or its reactive derivative is liquid, it can also be used as a solvent. The reaction is usually carried out at ambient temperature to about the boiling point of the solvent, although the reaction temperature is not specially restricted.

In this reaction, it is to be noted that, when a starting compound of general formula (II), in which Y is a valency bond and $R^2$ is a carboxy or esterified carboxy group, is used for the reaction, a compound (Ia') containing an alkanoyl radical is sometimes isolated in a form of the corresponding tautomeric hydroxymethylene compound of the formula (Ia") or of a mixture thereof, depending upon the conditions used for isolation. Furthermore, the hydroxymethylene isomer (Ia") can, if desired, be converted into the corresponding compound (Ia') by heating in a solvent, such as benzene or toluene, or the alkanoyl compound (Ia') can also be converted into the hydroxymethylene isomer (Ia") by treatment under basic conditions. The compound (Ia') and its isomer (Ia") are to be understood to be so-called keto-enol tautomers, as illustrated by the following equilibrium, and are to be regarded as being substantially the same compound:

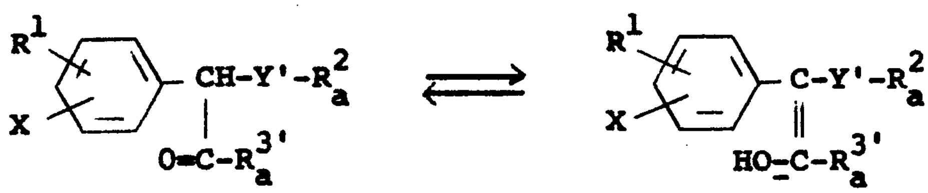

(Ia')    (Ia")

wherein $R^1$ and X have the same meanings as above, $R^2_a$ is a carboxy or esterified carboxy group, $R^{3'}_a$ is a hydrogen atom or a lower alkyl radical and Y' is a valency bond.

(2) Preparation B:

Compounds of the general formula:-

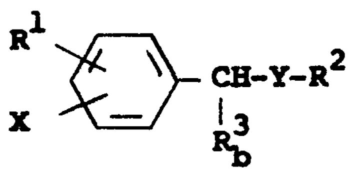

(Ib)

wherein $R^1$, $R^2$, X and Y have the same meanings as above and $R^3_b$ is hydroxymethyl, can be prepared by reacting a compound of general formula (II) with paraformaldehyde.

The reaction is preferably carried out in the presence of a strong base, such as is used in the above Preparation A. The reaction is usually carried out in a solvent, such as dimethyl sulphoxide, dimethyl formamide or some other conventional solvent, at ambient temperature. Compounds (Ib) can also be prepared by the following Preparation C.

(3) Preparation C:

Compounds of the general formula:-

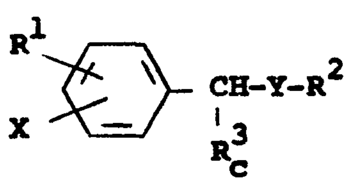

(Ic)

wherein $R^1$, $R^2$, X and Y have the same meanings as above, and $R_c^3$ is a hydroxy(lower)alkyl radical, can be prepared by reducing a compound of the general formula:-

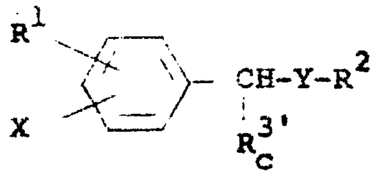

(IV)

wherein $R^1$, $R^2$, X and Y have the same meanings as above, and $R_c^{3'}$ is a lower alkanoyl radical.

The starting compounds (IV) can be prepared, for example, by Preparation A. It is to be noted that the starting compound (IV) includes the corresponding enol compound, as explained in Preparation A.

The reduction is carried out by conventional methods, for example, by using a reducing agent, such as an alkali metal borohydrate (e.g. lithium borohydrate, sodium borohydrate, potassium borohydrate or sodium cyanoborohydrate) or by catalytic reduction. The catalytic reduction is usually carried out in the presence of a conventional catalyst, such as Raney nickel, preferably at ambient temperature under atmospheric pressure and in a conventional solvent and the reduction using a reducing agent is usually carried out in a conventional solvent, preferably a polar solvent, such as water, tetrahydrofuran, methanol or ethanol, with cooling or at ambient temperature and optionally in the presence of a base, such as sodium hydroxide, sodium carbonate, potassium carbonate or sodium bicarbonate.

When a starting compound (IV) containing an esterified carboxy radical for $R^2$ is subjected to reduction, the esterified carboxy radical also is somtimes reduced simultaneously with the reduction of the lower alkanoyl group $R_c^{3'}$, depending upon the reaction conditions and/or the kind of reducing agent used to give a compound of the general formula:-

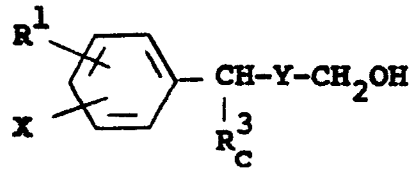

(Ic')

wherein $R^1$, $R^3_c$ , X and Y have the same meanings as above, it being understood that this is also within the scope of this process.

(4) Preparation D:

Compounds of the general formula:-

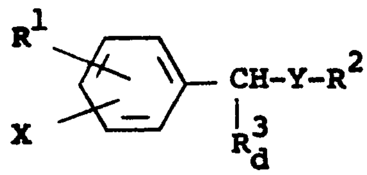

(Id)

wherein $R^1$, $R^2$, X and Y have the same meanings as above and $R^3_d$ is a lower alkoxy(lower)alkyl radical, can be prepared by reacting a compound of the general formula:-

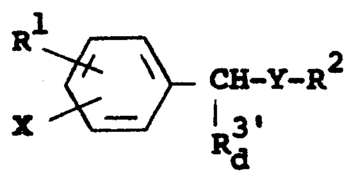

(V)

wherein $R^1$, $R^2$, X and Y have the same meanings as above and $R^{3'}_d$ is a hydroxy(lower)alkyl or acyloxy-(lower)alkyl radical, with an alkylating agent. The preferred alkylating agent can be a lower alkanol, lower alkyl halide (e.g. lower alkyl chloride, lower alkyl bromide or lower alkyl iodide), di(lower)alkyl sulphate, lower alkyl alkane or arene sulphonate (e.g. lower alkyl mesylate, lower alkyl benzene sulphonate, lower alkyl tosylate, etc.) and the like, in which the examples of the lower alkyl moieties can

be the same as those exemplified hereinbefore.

The reaction conditions are preferably selected in dependence upon the nature of the starting compound (V) employed, as follows.

Alkylation of a compound of general formula (V), in which Y is a valency bond and $R^2$ is a carboxy or esterified carboxy group, is preferably carried out by reacting with a lower alkanol in the presence of a strong base, such as an alkali metal alkoxide, and alkylation of a compound (V), in which $R^2$ is a hydroxymethyl radical, is preferably carried out by reaction with a conventional alkylating agent other than a lower alkanol, such as an alkyl halide, dialkyl sulphate or the like, in the presence of a strong base, such as an alkali metal hydride, alkali metal alkoxide or the like. In the former case, the reaction is more preferably carried out by using an excess of the lower alkanol, without any other solvent, with cooling and, in the latter case, the reaction is usually carried out in a solvent, such as dimethyl formamide, tetrahydrofuran, dimethyl sulphoxide, monoglyme, hexamethylphosphoryltriamide or other conventional solvent, with cooling or at ambient temperature. Furthermore, in this reaction, it is to be noted that when the alkyl moiety of the alcohol to be used as a reagent in the reaction is different from the ester part of the esterified carboxy radical for $R^2$ of the starting compound (V) (depending upon the reaction conditions, e.g. nature of the alkylating agent and the base, the presence of water and/or the reaction temperature, etc.), in the former case, transesterification usually takes place as a side reaction so that the ester part of the esterified carboxy for $R^2$ is converted into the different ester part and/or hydrolysis of the esterified carboxy for $R^2$ sometimes

also takes place as a side reaction so that the esterified carboxy for $R^2$ is converted into the corresponding free carboxylic acid or its salt.

Furthermore, when the alkylating agent is used in excess in the latter case, the hydroxymethyl radical for $R^2$ of the starting compound (V) is sometimes alkylated to give a compound having a lower alkoxymethyl radical for $R^2$.

Furthermore, compound of the general formula:-

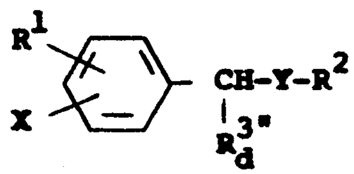

(Id')

wherein $R^1$, $R^2$, X and Y have the same meanings as above and $R_d^{3''}$ is a lower alkoxymethyl radical, can be prepared by treating a compound of the general formula:-

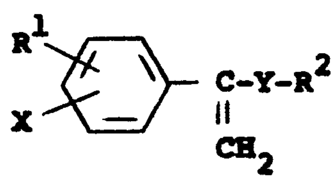

(VI)

wherein $R^1$, $R^2$, X and Y have the same meanings as above, in the same manner as described above.

Of the starting compounds (VI), methyl 2-(4-cyclohexylphenyl)acrylate is known and can be prepared, for example, by the method described in Chemical Abstracts 66, 10765 d/1967 and the other compound (VI) can also be prepared according to this method.

(5) Preparation E:

Compounds of the general formula:-

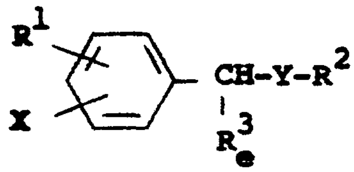

(Ie)

wherein $R^1$, $R^2$, X and Y have the same meanings as above and $R_e^3$ is an acyloxy(lower)alkyl radical, can be prepared by reacting a compound of the general formula:-

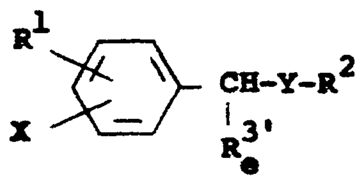

(VII)

wherein $R^1$, $R^2$, X and Y have the same meanings as above and $R_e^{3'}$ is a hydroxy(lower)alkyl radical, with an acylating agent.

The acylating agent can be a carboxylic or sulphonic acid having an acyl moiety as illustrated hereinbefore, or a salt or a reactive derivative thereof or a haloformic acid ester.

Examples of salts of carboxylic or sulphonic acids include salts with inorganic or organic bases, such as alkali metal salts (e.g. sodium salts, potassium salts, etc.), alkaline earth metal salts (e.g. calcium salts, magnesium salts, etc.), organic base salts (e.g. triethylamine salts, pyridine salts, etc.) and the like.

Examples of reactive derivatives of carboxylic sulphonic acids include acid halides, acid azides, acid anhydrides, activated amides and activated esters, such as those illustrated for the lower alkanoic acids (III) in the Preparation A.

The reaction is usually carried out in a conventional solvent, such as methylene chloride, chloroform, benzene, diethyl ether, tetrahydrofuran, pyridine or

the like, and preferably in the presence of an organic or inorganic base, such as an alkali metal bicarbonate (e.g. sodium or potassium bicarbonate, etc.), an alkali metal hydroxide (e.g. sodium or potassium hydroxide, etc.), or an organic base (e.g. triethylamine, dimethylbenzylamine, N-methylmorphorine, N-methylpiperidine, pyridine, etc.). The reaction temperature is not particularly limited but the reaction is preferably carried out with cooling or at an ambient temperature. When the acylating agent is used in the form of a free acid or in salt form, the reaction is preferably carried out in the presence of a conventional condensation agent, such as a carbodiimide compound (e.g. N,N'-dicyclohexylcarbodiimide, N-cyclohexyl-N'-morpholinoethylcarbodiimide, N-cyclohexyl-N'-(4-diethylaminocyclohexyl)-carbodiimide, N,N'-diethylcarbodiimide, N,N'-diisopropylcarbodiimide, N-ethyl-N'-(3-dimethylaminopropyl)-carbodiimide, etc.), N,N'-carbonyl-bis-(2-methylimidazole), pentamethyleneketone-N-cyclohexylimine, diphenylketene-N-cyclohexylimine, an alkoxyacetylene, a 1-alkoxy-1-chloroethylene, a trialkyl phosphite, ethyl polyphosphate, isopropyl polyphosphate, phosphorus oxychloride, phosphorus trichloride, thionyl chloride, oxalyl chloride, triphenylphosphine, N-ethylbenzisoxazolium salt, N-ethyl-5-phenyl-isoxazolium-3'-sulphonate, 1-(p-chlorobenzenesulphonyloxy)-6-chloro-1H-benzotriazole, a Vilsmeier reagent (e.g. (chloromethylene)-dimethylammonium chloride, a compound formed by the reaction of dimethylformamide with phosphorus oxychloride, etc.) or the like.

In this reaction, it is to be noted that, when a starting compound (VII) having a hydroxymethyl radical for $R^2$ is used for the reaction, the hydroxy function of the hydroxymethyl radical for $R^2$ of the starting compound (VII) can also be acylated to give

the corresponding diacyl compound (i.e. the compound (Ie), in which $R^2$ is acyloxymethyl, depending upon the amount of acylating agent used. It is to be understood that this mode of reaction is also included within the scope of Preparation E.

(6) <u>Preparation F</u>:

Compounds of the general formula:-

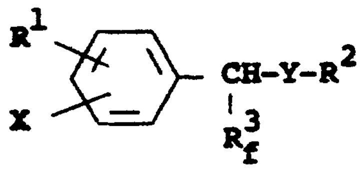

(If)

wherein $R^1$, $R^2$, X and Y have the same meanings as above and $R_f^3$ is an amino(lower)alkyl, lower alkylamino(lower)alkyl or di(lower)alkylamino(lower)alkyl radical, in which the lower alkyl moieties of the di(lower)alkylamino group can also be joined together to form a ring containing the nitrogen atom, can be prepared by reacting a compound of the general formula:-

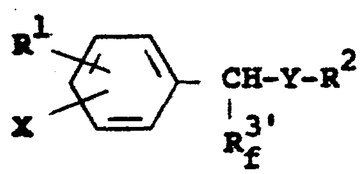

(VIII)

wherein $R^1$, $R^2$, X and Y have the same meanings as above and $R_f^{3'}$ is an acyloxy(lower)alkyl radical, with ammonia or an amine compound selected from alkylamines, di(lower)alkylamines and saturated heterocyclic imine compounds containing an imino group, or by reacting a compound of the general formula:-

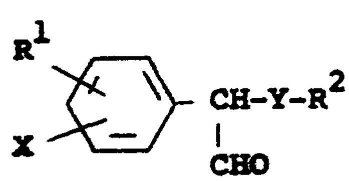

(IX)

wherein $R^1$, $R^2$, X and Y have the same meanings as above, with ammonia or an amine compound selected from lower alkylamines, di(lower)alkylamines and saturated heterocyclic imine compounds containing an imino group, in the presence of a reducing agent.

The lower alkyl moiety of the lower alkylamines and di(lower)alkylamines employed can be the same as those illustrated hereinbefore.

Examples of saturated heterocyclic imine compounds containing an imino group include aziridine, azetidine, pyrrolidine and piperidine.

The reaction of a compound (VIII) with ammonia ot an amine is preferably carried out by using an excess amount thereof, for example two or more mole equivalents of ammonia or of the amine.

The reaction is also preferably carried out in the presence of a strong base, as illustrated in the case of Preparation A. The reaction is usually carried out in a solvent, such as water, methanol, ethanol, tetrahydrofuran, dioxan, N,N-dimethylformamide or some other conventional solvent, with cooling or at ambient temperature.

Examples of the reducing agent to be employed for the reaction of the compound (IX) with ammonia or an amine include those exemplified in Preparation C.

The reaction of a compound (IX) with ammonia or an amine is usually carried out in a solvent, such as tetrahydrofuran, methanol, ethanol or n-propanol, with cooling or at ambient temperature. The reaction employing a compound (IX) can especially be used to prepare a compound having an aminomethyl, lower alkylaminomethyl or di(lower)alkylaminomethyl radical, in which the lower alkyl moieties of the di(lower)alkylamino group can be joined together to form a saturated heterocyclic radical containing a nitrogen atom, as $R^3_f$ in formula (If).

(7) Preparation G:

Compounds of the general formula:-

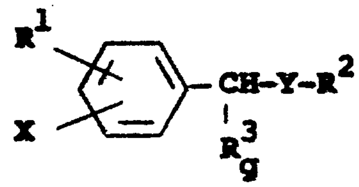

(Ig)

wherein $R^1$, $R^2$, X and Y have the same meanings as above and $R_g^3$ is an acylamino(lower)alkyl radical, can be prepared by reacting a compound of the general formula:-

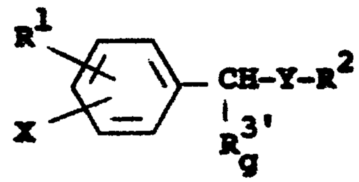

(X)

wherein $R^1$, $R^2$, X and Y have the same meanings as above and $R_g^{3'}$ is an amino(lower)alkyl radical, with an acylating agent.

The acylating agents to be employed in this process can be the same as those exemplified in Preparation E.

When a free carboxylic or sulphonic acid or a salt thereof is used as acylating agent, the reaction is preferably carried out in the presence of a conventional condensation agent as exemplified in Preparation E. Furthermore, when a reactive derivative of a carboxylic or sulphonic acid or a haloformic acid ester is used as acylating agent, the reaction is preferably carried out in the presence of an inorganic or organic base, such as sodium bicarbonate, sodium carbonate, sodium hydroxide, triethylamine, dimethylbenzylamine, N-methylmorphorine, N-methylpiperidine or pyridine. The reaction conditions are substantially the same as those illustrated in

Preparation E, i.e. the reaction is usually carried out in a conventional solvent, such as water, methylene chloride, chloroform, carbon tetrachloride, benzene or diethyl ether, while cooling. A liquid condensation agent or a liquid base can also be used as solvent.

(8) Preparation H:

Compounds of the general formula:-

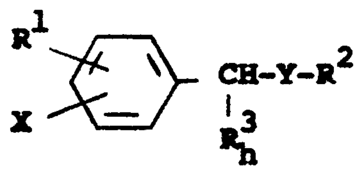

(Ih)

wherein $R^1$, $R^2$, X and Y have the same meanings as above and $R_h^3$ is an acyl(lower)alkyl radical, can be prepared by reacting a compound of the general formula:-

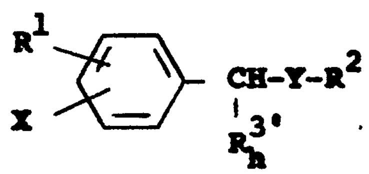

(XI)

wherein $R^1$, $R^2$, X and Y have the same meanings as above and $R_h^{3'}$ is a hydroxy(lower)alkyl or acyloxy(lower)alkyl radical, or a compound of the general formula:-

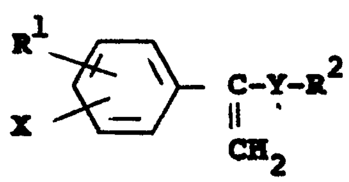

(XII)

wherein $R^1$, $R^2$, X and Y have the same meanings as above, with nitro(lower)alkane or aryl-substituted nitro-(lower)alkane in the presence of a strong base, followed by treating the resultant product with a mineral acid or an oxidising agent.

Examples of nitro(lower)alkanes include straight-

chained nitro(lower)alkanes, such as nitromethane, nitroethane, nitropropane, nitrobutane, nitropentane, nitrohexane or the like, and aryl-substituted nitro-(lower)alkane include phenyl-substituted straight-chained nitro(lower)alkane such as 1-nitro-2-phenylethane, phenyl-nitromethane and the like.

Examples of strong bases to be used in this process include inorganic bases, such as alkali metal hydrides (e.g. sodium hydride, potassium hydride and lithium hydride), alkali metal alkoxides (e.g. sodium alkoxides and potassium alkoxides and the like.

The reaction of the compound (XIII) or (XII) with the nitro(lower)alkane or aryl-substituted nitro-(lower)alkane is usually carried out in a solvent, such as methanol, ethanol, tetrahydrofuran or other conventional solvent, at ambient temperature. The product thus obtained can conveniently be treated with a mineral acid or an oxidising agent without isolation and/or purification.

Examples of the mineral acids to be used include hydrochloric acid and sulphuric acid.

Examples of the oxidising agent to be used include conventional ones, such as permanganates (e.g. potassium permanganate), chromates (e.g. chromic acid) and the like.

The treatment of the resultant product with the mineral acid or oxidising agent can be carried out conventionally by introducing a mineral acid or an oxidising agent into the reaction mixture obtained above, with cooling.

When a compound of general formula (XII) is used in this Preparation H, a compound (Ih) in which the radical $R_h^3$ is acylmethyl radical is obtained as the final product.

In the aforementioned Preparations D and F, the reaction of the hydroxymethyl and acyloxymethyl compounds (i.e. a compound (V) in which $R_d^{3'}$ is hydroxymethyl

or acyloxymethyl and a compound (VIII) in which $R_f^{3'}$ is acyloxymethyl) sometimes proceeds <u>via</u> a compound of general formula (XII). Therefore, the compound (XII) can also be used as starting material in these Preparations.

(9) <u>Preparation I</u>:

Compounds of the general formula:-

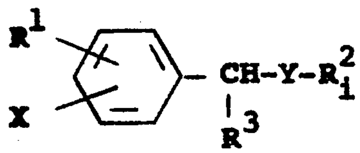

(Ii)

wherein $R^1$, $R^3$, X and Y have the same meanings as above and $R_i^2$ is a carboxy radical, or a salt thereof, can be prepared by hydrolysing a compound of the general formula:-

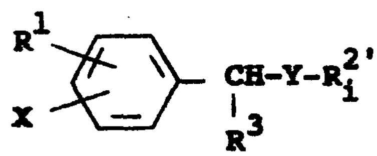

(XIII)

wherein $R^1$, $R^3$, X and Y have the same meanings as above and $R_i^{2'}$ is an esterified carboxy radical.

The hydrolysis is usually carried out by treating a compound (XIII) with a base or an acid in water or an aqueous mixture of a hydrophilic organic solvent, such as methanol, ethanol, <u>n</u>-propanol, isopropanol, tetrahydrofuran, acetone or the like.

Examples of the bases include inorganic and organic bases, such as alkali metal hydroxides (e.g. sodium hydroxide, potassium hydroxide, etc.), alkaline earth metal hydroxides (e.g. barium hydroxide, calcium hydroxide, magnesium hydroxide, etc.) and alkali metal alkoxides (e.g. sodium methoxide, sodium ethoxide, etc.).

Examples of the acids include inorganic acids, such as hydrochloric acid, hydrobromic acid and sulphuric acid, and organic acids, such as formic acid, trifluoroacetic acid, methanesulphonic acid and <u>p</u>-toluenesulphonic acid.

The reaction conditions used depend upon the nature of starting material (XIII) and the nature of the reagent, i.e. acid or base, and the reaction is usually carried out with cooling, at ambient temperature or with warming or heating. When compounds having a lower alkoxy(lower)alkyl radical for $R^3$ in general formula (XIII), in which the lower alkoxy moiety contains more than 2 carbon atoms, are employed as starting material, the hydrolysis is preferably carried out by using an acid, such as formic acid, methanesulphonic acid, p-toluenesulphonic acid or sulphuric acid, or a mixture thereof.

The compound (Ii) can also be prepared by reacting a compound of the general formula:-

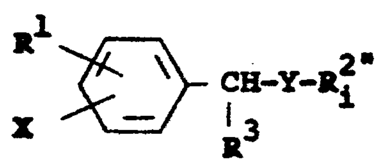

(XIV)

wherein $R^1$, $R^3$, X and Y have the same meanings as above and $R_i^{2"}$ is an acyloxymethyl radical, with a diester of malonic acid in the presence of a base and then hydrolysing the resultant compound of the general formula:-

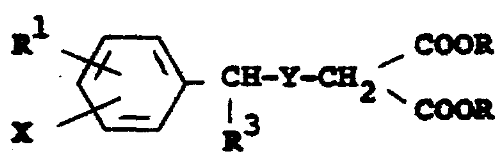

(XV)

wherein $R^1$, $R^3$, X and Y have the same meanings as above and COOR is an esterified carboxy group, and finally decarboxylating the resultant dicarboxylic acid.

The hydrolysis and decarboxylation reactions of this method can be carried out in a conventional manner, using the so-called malonic ester synthesis. According to this method, a compound having one more

methylene radical than the starting material can be obtained.

(10) Preparation J:

Compounds of the general formula:-

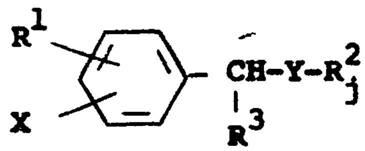

(Ij)

wherein $R^1$, $R^3$, X and Y have the same meanings as above and $R^2_j$ is a hydroxymethyl radical, can be prepared by reducing a compound of the general formula:-

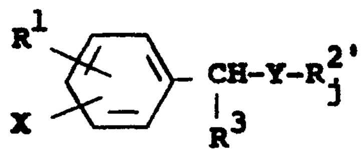

(XVI)

wherein $R^1$, $R^3$, X and Y have the same meanings as above and $R^{2'}_j$ is a carboxy or esterified carboxy radical.

The reduction of the compounds (XVI) is usually carried out by using a hydride type of reducing agent, such as lithium aluminium hydride, aluminium hydride, lithium trimethoxyaluminium hydride or diborane, in a solvent, such as diethyl ether, tetrahydrofuran, benzene or toluene. The reduction can also be carried out by using sodium borohydride in the presence of a Lewis acid, such as aluminium chloride.

The reaction is usually carried out with cooling or at a somewhat elevated temperature but the reaction temperature is not particularly restricted.

When preparing compounds ($I_j$), wherein $R^3$ is a lower alkanoyl or acyl(lower)alkyl radical, the corresponding carbonyl function of the acyl(lower)alkyl radical of the starting compound (XVI) is to be protected prior to carrying out this reduction and thereafter the protected carbonyl function is transformed

into the carbonyl function. The protection of the carbonyl function can conveniently be carried out by reacting the compound (XVI) with a hydroxy compound, such as ethylene glycol, in the presence of an acid in a conventional manner for well known acetalisations.

Transformation of the carbonyl function from the protected carbonyl group is usually carried out by subjecting the reduction product to hydrolysis or transacetalisation. The hydrolysis is preferably carried out by treating the product in the presence of an inorganic acid, such as hydrochloric acid or sulphuric acid, or with an organic acid, such as formic acid, trifluoroacetic acid or p-toluenesulphonic acid, in conventional manner, and the transacetalisation reaction is usually carried out by treating the product with an excess amount of a ketone, such as acetone, in the presence of a catalytic amount of acid, as mentioned above, in a conventional manner.

When the above-mentioned carbonyl function of the starting compound (XVI) is not protected, the carbonyl function is sometimes reduced to give a diol compound (Ij), wherein $R^3$ is a hydroxy(lower)alkyl radical. These cases are also included within the scope of this Preparation.

(11) Preparation K:

Compounds of the general formula:-

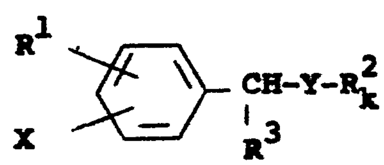

(Ik)

wherein $R^1$, $R^3$, X and Y have the same meanings as above and $R_k^2$ is an acyloxymethyl radical, can be prepared by reacting the compound of the general formula:-

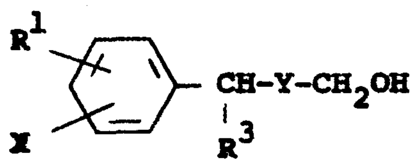

(Ij)

wherein $R^1$, $R^3$, X and Y have the same meanings as above, with an acylating agent.

The acylating agent to be used in this reaction includes those illustrated in Preparation E.

The acylation can be carried out in substantially the same manner as described above in Preparation E. In this reaction, it is to be noted that the product (Ik), in which $R^3$ is lower alkanoyl or acyl(lower)-alkyl, is also isolated from the reaction mixing given by the reaction using the compound (Ij), in which the carbonyl function of lower alkanoyl or acyl(lower)-alkyl for $R^3$ is protected, as the starting compound. This case is also included within the scope of this Preparation.

(12) Preparation L:

Compounds of the general formula:-

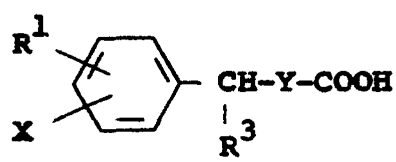

(Iℓ)

wherein $R^1$, $R^3$, X and Y have the same meanings as above, and the salts thereof can be prepared by oxidising a compound of the general formula:-

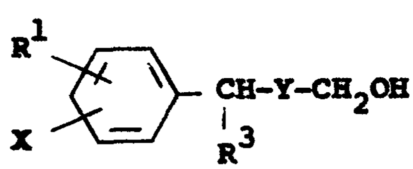

(Ij)

wherein $R^1$, $R^3$, X and Y have the same meanings as above.

The oxidation of the compounds (Ij) is usually carried out by treating the compound Ij) with an oxidising agent, such as chromic acid or potassium permanganate, in a solvent, such as acetic acid, acetone or water, or in a mixture thereof.

The reaction is usually carried out with cooling but the reaction temperature is not restricted thereto.

(13) Preparation M:

Compounds of the general formula:-

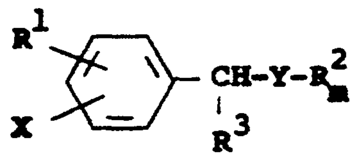

(Im)

wherein $R^1$, $R^3$, X and Y have the same meanings as above and $R^2_m$ is an esterified carboxy radical, can be prepared by esterifying a compound of the general formula:-

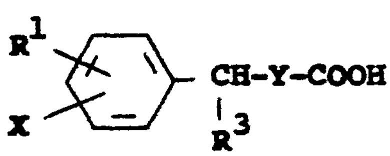

($I\ell$)

wherein $R^1$, $R^3$, X and Y have the same meanings as above.

The esterification is carried out by reacting the compound ($I\ell$), a reactive carboxy derivative thereof or a salt thereof with an esterifying agent.

The preferred reactive derivatives of the carboxy group of the compounds ($I\ell$) include those illustrated for acylating agents in Preparation E, for example, acid halides, acid anhydrides, activated amides, activated esters and the like.

The esterifying agent can be a hydroxy compound and a reactive equivalent thereof. Examples of the hydroxy compound include substituted and unsubstituted alcohols, examples of which are given in the case of

the ester moiety described in the illustrations of the esterified carboxy radical for $R^2$. Preferred reactive equivalents of the hydroxy compounds include conventional ones, such as the corresponding halides, alkanesulphonates, arenesulphonates and salts of the hydroxy compound, diazoalkanes, diazoaralkanes and the like.

The reaction can be carried out in the presence or absence of a solvent, such as N,N-dimethylformamide, dimethylsulphoxide or any other solvent which does not adversely influence the reaction, with cooling or heating. This reaction is preferably carried out in the presence of an inorganic or organic base, such as one of those exemplified in Preparation E. A liquid hydroxy compound or base can also be used as the solvent in this reaction.

If the free carboxylic acid form of the starting compound (Ii) or a salt and/or free hydroxy derivative thereof is employed in this preparation, the reaction is preferably carried out in the presence of a conventional condensing agent, such as an inorganic or organic acid (e.g. hydrochloric acid, sulphuric acid, p-toluenesulphonic acid, etc.) or of one of those particularly illustrated hereinbefore in Preparation E.

Although the reaction and/or post-treatment in the Preparations A to M may be sometimes accompanied by side reactions other than those mentioned hereinbefore, it is to be understood that the products obtained by such side reactions are also included within the scope of the compounds (I) of the present invention.

The compound (I) may, if desired, be converted into a pharmaceutically-acceptable salt, such as an alkali metal salt (e.g. a sodium salt or potassium salt), an alkaline earth metal salt (e.g. a calcium

salt or a magnesium salt), an organic amine salt (e.g. a triethylamine salt or a dicyclohexylamine salt), an inorganic acid salt (e.g. a hydrochloride, hydrobromide or sulphate), an organic acid salt (e.g. a tartrate or maleate) or an amino acid salt (e.g. an arginine salt, an aspartate or a glutamate), by conventional methods.

The following pharmacological test data show that the compounds (I) of the present invention exhibit an anti-inflammatory activity and are useful as anti-inflammatory agents for treating inflammation in animals and mammals.

Test method (1):

Ten male 5 week old Hartley rats, each weighing about 350 g., were used per group. The backs of each animal were depilated 24 hours before the test. An adhesive tape with 3 small holes of 9 mm. diameter was placed on the depilated skin and then the animal was exposed to ultra-violet radiation from an ultra-violet lamp (500 W, manufactured by Engelhard Hanovia Inc.) at a distance of 13 cm. for 80 seconds. Two hours later, the degree of erythema was estimated on the basis of the following scores:

1.0 : eryhthema with clear border,
0.5 : erythema with unclear border,
0.0 : scarcely any erythema.

The compounds were regarded as being effective when the total of 3 points was below 1.5.

The valuation was determined as follows:

| Score | number of rats estimated as effective | the dose administered |
| --- | --- | --- |
| - | 0 | 500 mg/kg |
| ± | 1 - 7 | 500 mg/kg |
| +1 | 8 - 10 | 500 mg/kg |
| +2 | 8 - 10 | 125 mg/kg |
| +3 | 8 - 10 | 32 mg/kg |
| +4 | 8 - 10 | 8 mg/kg |
| +5 | 8 - 10 | 2 mg/kg |

Each dosage of the test compound was administered orally in a suspension form in 20 ml. of 0.5% aqueous methyl cellosolve aqueous solution. Half of the test sample was administered one hour before the radiation and the remaining half of the test solution was administered

just after radiation. The test results obtained are given in the following Table 1:

Table 1

| Test compound obtained in Example | Score |
|---|---|
| 2 | +4 |
| 4-(3) | +5 |
| 5-(3) | +4 |
| 7-(1) | +4 |
| 7-(2) | +2 |
| 8 | +3 |
| 9 | +4 |
| 11 | +2 |
| 13 | +4 |
| 14-(3) | +1 |
| 15-(2) | +3 |
| 16-(3) | +3 |
| 17-(2) | +3 |
| 20 | +1 |
| 21-(2) | +2 |
| 22 | +1 |
| 23 | +5 |
| 24-(2) | +4 |

<u>Test Method (2)</u> :

The stomach was removed from Sprague-Dawley rats, weighing about 180 g., after the animals were fasted overnight. A strip of stomach fundus was suspended under initial tension of 0.6 g. in a 10 ml. organ bath containing Tyrode solution.

Arachidonic acid (1.0 x $10^{-5}$ g/ml.) was employed as the

spasmogen. Several doses of the test compound were added to the individual bath fluid 15 minutes before the addition of arachidonic acid. The value of contraction induced by arachidonic acid was measured and plotted as a dose-activity curve. The $ED_{50}$ value of each test compound was obtained by interpolation from the dose-activity curve. The results obtained are given in the following Table 2.:-

Table 2.

| Test compound obtained in Example | $ED_{50}$ (g./ml.) |
|---|---|
| 2 | $2.6 \times 10^{-6}$ |
| 4-(3) | $8.4 \times 10^{-8}$ |
| 5-(3) | $1.2 \times 10^{-7}$ |
| 7-(1) | $1.2 \times 10^{-6}$ |
| 7-(2) | $5.0 \times 10^{-7}$ |
| 9 | $2.6 \times 10^{-7}$ |
| 12-(2) | $2.6 \times 10^{-6}$ |
| 14-(3) | $2.2 \times 10^{-5}$ |
| 15-(2) | $3.2 \times 10^{-6}$ |
| 17-(2) | $1.3 \times 10^{-6}$ |
| 24-(2) | $4.2 \times 10^{-6}$ |
| Reference compound (aspirin) | $3.1 \times 10^{-6}$ |

As can be seen from the above test results, the compounds (I) of the present invention are useful as anti-inflammatory agent.

The active compound is usually administered at a dosage of 10 to 500 mg., 1 to 4 times a day in the form of preparations, such as tablets, granules, powders, capsules, syrups, injections or suppositories. However, the dosage can be increased or decreased, depending upon

the age, weight or condition of the patient or upon the method of administration. The compositions can be prepared in a conventional manner by using conventional solid or liquid carriers and additives.

The following Examples are give for the purpose of illustrating the present invention:-

Example 1

(1) A solution of 28.8 mg. sodium in 2.5 ml. methanol was added dropwise to a suspension of 11.6 g. methyl p-cyclohexylphenylacetate and 1.5 g. paraformaldehyde in 30 ml. dimethyl sulphoxide over the course of 10 minutes, whereafter the reaction mixture was stirred at ambient temperature for 5 minutes. 0.3 ml. Acetic acid were added dropwise to the reaction mixture, whereafter ethyl acetate and cold water were added thereto. The mixture was shaken and the organic layer was then separated, successively washed with water, with a saturated aqueous solution of sodium bicarbonate and again with water, dried over anhydrous magnesium sulphate and then evaporated to dryness under reduced pressure. The oily residue was dissolved in n-hexane and the solution left to stand overnight. The precipitated crystals were filtered off with suction and washed with n-hexane to give 6.72 g. of colourless crystals of methyl p-cyclohexyl tropate. A further 1.17 g. of the same product was recovered from the mother liquor by concentration and crystallization. Total yield 7.89 g.; m.p. 83.5 - 85.5°C.

I.R. $\nu_{max.}^{Nujol}$ : 3250, 1735 $cm^{-1}$

N.M.R. $\delta_{ppm}^{CDCl_3}$: 1.10 to 2.10 (10H, m)
2.26 to 2.76 (2H, broad s)
3.64 (3H, s)
3.69 to 4.23 (3H, m)
7.10 to 7.33 (4H, m)

(2) 990.6 mg. Tosyl chloride was added all at once to a cold solution of 1.048 g. methyl p-cyclohexyl tropate in 4 ml. pyridine and the reaction mixture was first stirred for 30 minutes while cooling with ice and then overnight at ambient temperature. Ethyl acetate was added to the reaction mixture, which was then washed successively with water, 5% hydrochloric acid, water, a

saturated aqueous solution of sodium bicarbonate and water, dried over anhydrous magnesium sulphate and then evaporated to dryness under reduced pressure. The oily residue was crystallized by drying in a desiccator to give 1.598 g. of crystals of methyl 2-(4-cyclohexylphenyl)-3-tosyloxypropionate.

I.R. $\nu_{max}^{Nujol}$ : 1730, 1365, 1170 $cm^{-1}$

N.M.R. $\delta_{ppm}^{CDCl_3}$ : 1.05 to 2.15 (10H, m)
2.41 (3H, s)
3.59 (3H, s)
3.69 to 4.71 (3H, m)
6.92 to 7.35 (4H, m)
7.28 (2H, d, J=8Hz)
7.71 (2H, d, J=8Hz)

Example 2

A solution of 3.275 g. methyl p-cyclohexyl tropate, obtained in the Example 1-(1), in 20 ml. tetrahydrofuran was added dropwise to a suspension of 3.944 g. barium hydroxide octahydrate in 40 ml. water, with stirring, over the course of 15 minutes, while cooling with ice. The reaction mixture was stirred, with ice-cooling, for 3 hours and at ambient temperature for a further 5 hours and then left to stand overnight at ambient temperature. The precipitate was filtered off with suction, washed with a mixture of tetrahydrofuran and water (1:1 v/v) and then suspended in diethyl ether and water. The suspension was adjusted to pH 1 with 10% hydrochloric acid, while stirring under cooling with ice, the ether layer was separated off, washed with water, dried over anhydrous magnesium sulphate and then evaporated under reduced pressure. The resultant crystals were recrystallized from a mixture of acetone and diethyl ether to give 1.752 g. p-cyclohexyltropic acid; m.p. 159 - 160.5°C.

Analysis:

calc. for $C_{15}H_{20}O_3$ : C 72.55%; H 8.12%
found : 72.63%; 8.23%

I.R. $\nu_{max}^{Nujol}$ : 3500, 1715 $cm^{-1}$

N.M.R. $\delta_{ppm}^{DMSO-d_6}$ : 1.10 to 2.03 (10H, m)
2.40 (1H, broad s)
3.40 to 4.03 (3H, m)
7.20 (4H, m)

Example 3

(1) A solution of 23.2 g. methyl p-cyclohexylphenyl acetate in 7.2 g. methyl formate was added dropwise to a suspension of 50% sodium hydride (5.76 g.) in 150 ml. diethyl ether over the course of 25 minutes at 25 - 30°C., while stirring, and the reaction mixture then stirred at ambient temperature for 3 hours. Ice-water was added to the reaction mixture and the aqueous layer was separated off and washed with diethyl ether, 7.89 g. of unreacted starting material being recovered from the washings. The aqueous layer was mixed with diethyl ether and adjusted to pH 1 with 10% hydrochloric acid, while stirring under cooling with ice. The ether layer was separated off, washed with water, dried over anhydrous magnesium sulphate and then evaporated to dryness under reduced pressure. The residue was triturated with n-hexane and the resultant precipitate was filtered off to give 5.959 g. methyl 2-(p-cyclohexylphenyl)-2-hydroxymethyleneacetate. The mother liquor was evaporated to dryness under reduced pressure to give crude methyl 2-(p-cyclohexylphenyl)-2-formyl acetate. This formyl compound was converted into the above-mentioned hydroxymethylene compound as follows:

A cold solution of the crude formyl compound in a mixture of 1N aqueous sodium hydroxide and diethyl ether was adjusted to pH 1 with 10% hydrochloric acid. The diethyl ether layer was separated off, washed with water,

dried over anhydrous magnesium sulphate and then evaporated under reduced pressure. The residue was triturated with n-hexane to give 4.434 g. of crystalline methyl 2-(p-cyclohexylphenyl)-3-hydroxyacrylate which was identified with a specimen of the compound obtained as above. 6.42 g. Methyl 2-(p-cyclohexylphenyl)-2-formyl acetate were recovered from the trituration mother liquor by evaporating to dryness under reduced pressure. Methyl 2-(p-cyclohexylphenyl)-3-hydroxyacrylate has the following characteristics:

I.R. $\nu_{max}^{Nujol}$ : 3150, 1650 (sh), 1620 $cm^{-1}$

N.M.R. $\delta_{ppm}^{CDCl_3}$ : 1.17 to 2.07 (10H, m)
2.26 to 2.76 (1H, broad s)
3.73 (3H, s)
5.92 (1H, d, J=14Hz)
7.10 to 7.40 (4H, m)
7.71 (1H, d, J=14Hz)

Methyl 2-(p-cyclohexylphenyl)-2-formyl acetate has the following characteristics:

I.R. $\nu_{max}^{liquid\ film}$ : 1700, 1660, 1600 $cm^{-1}$

N.M.R. $\delta_{ppm}^{CDCl_3}$ : 1.17 to 2.06 (10H, m)
2.25 to 2.74 (1H, broad s)
3.76 (3H, s)
7.10 to 7.32 (4H, m)
7.23 (1H, d, J=13Hz)
12.04 (1H, d, J=13Hz)

(2) A solution of 6.032 g. methyl 2-(p-cyclohexylphenyl)-3-hydroxyacrylate in 70 ml. methanol was shaken with hydrogen at atmospheric pressure in the presence of 15 ml. Raney nickel at ambient temperature. After the calculated amount of hydrogen (1 mole equivalent) had been adsorbed, the Raney nickel

was filtered off and the filtrate was evaporated to dryness under reduced pressure to give 5.93 g. crystalline methyl p-cyclohexyl tropate; m.p. 80.5 - 82.5°C.

I.R. $\nu_{max}^{Nujol}$ : 3250, 1730 $cm^{-1}$

N.M.R. $\delta_{ppm}^{CDCl_3}$ : 1.05 to 2.02 (10H, m)
2.25 to 2.68 (2H, broad s)
3.59 (3H, s)
3.56 to 4.18 (3H, m)
7.02 to 7.26 (4H, m)

(3) 2.97 g. Acetic anhydride were added dropwise to a solution of 5.45 g. methyl p-cyclohexyl tropate in 6 ml. pyridine over the course of 45 minutes, with stirring and cooling with ice. The reaction mixture was stirred at the same temperature for 2 hours. Diethyl ether was added to the reaction mixture and the mixture was washed successively with ice-cold 10% hydrochloric acid, water, a saturated aqueous solution of sodium bicarbonate and water, dried over anhydrous magnesium sulphate and then evaporated to dryness under reduced pressure. The oily residue was dried in vacuo to give 6.1 g. of crystalline methyl 2-(p-cyclohexylphenyl)-3-acetoxypropionate (6.1.g.)

I.R. $\nu_{max}^{liquid\ fild}$ : 1735 $cm^{-1}$

N.R.M. $\delta_{ppm}^{CDCl_3}$ : 1.10 to 2.05 (10H, m)
2.07 (3H, s)
2.20 to 2.66 (1H, m)
3.74 (3H, s)
3.58 to 4.76 (3H, m)
7.30 to 7.40 (4H, m)

Example 4

(1) About 1/3 volume of a solution of 66.7 mg. sodium in 5.8 ml. methanol was added dropwise to a

solution of 15.457 g. methyl 2-(3-chloro-4-cyclohexylphenyl)acetate and 1.74 g. paraformaldehyde in 30 ml. dimethyl sulphoxide over the course of 5 minutes. After stirring the reaction mixture for 5 minutes, 0.3 ml. acetic acid was added dropwise thereto and further ethyl acetate and water were added. After shaking, the ethyl acetate layer was separated off. The remaining aqueous layer was extracted with ethyl acetate and the extract was combined with the ethyl acetate layer, washed successively with water, a saturated aqueous solution of sodium bicarbonate and water, dried over anhydrous magnesium sulphate and then evaporated to dryness under reduced pressure to give 18.12 g. of oily methyl m-chloro-p-cyclohexyltropate. This compound was used for the following reaction without purification.

(2) A solution of 0.23 g. sodium in 10 ml. methanol was added dropwise to a solution of 8.895 g. methyl m-chloro-p-cyclohexyltropate, obtained as above, in 50 ml. methanol over the course of 20 minutes under reflux and with stirring, whereafter the reaction mixture was stirred for 2 hours under the same conditions. After distilling off the methanol under reduced pressure, diethyl ether and water were added to the residue. The mixture was adjusted to pH 1 with 10% hydrochloric acid and the ether layer was separated, washed with water and dried over anhydrous magnesium sulphate. The solution was treated with an ethereal solution of diazomethane and evaporated to dryness under reduced pressure. The residue was chromatographed on a silica gel column, elution being carried out with chloroform after washing with a mixture of n-hexane and benzene. The eluate was treated in a conventional manner to give 3.923 g. of oily methyl 2-(3-chloro-4-cyclohexylphenyl)-3-methoxypropionate.

I.R. $\nu_{max.}^{liq.\ film}$ : 1735 $cm^{-1}$

N.M.R. $\delta_{ppm}^{CDCl_3}$ : 1.10 to 2.07 (10H, m)
2.69 to 3.05 (1H, m)
3.28 (3H, s)
*3.43 to 4.03 (3H, m)
3.61 (3H, s)
7.10 to 7.36 (3H, m)

(3) 2.484 g. of the methyl 2-(3-chloro-4-cyclohexylphenyl)-3-methoxypropionate thus obtained were added to 50 ml. methanol and 7 ml. 10% aqueous sodium hydroxide solution and the reaction mixture was stirred at 70 - 75°C. for 1 hour. Methanol was distilled off under reduced pressure and ethyl acetate and water were added to the residue. The mixture was adjusted to pH 1 with 10% hydrochloric acid, while stirring and cooling with ice, and the ethyl acetate layer was separated off, washed with an aqueous solution of sodium chloride, dried over anhydrous magnesium sulphate and then evaporated to dryness under reduced pressure. The oily residue was added to a solution of 0.3 g. sodium hydroxide in 10 ml. methanol and the mixture was evaporated to dryness under reduced pressure. The residue was dissolved in warm ethanol and a small amount of acetone was added to the solution. The mixture was left to stand at ambient temperature and the resultant precipitate was filtered off with suction and dried to give 1.772 g. of crystalline sodium 2-(3-chloro-4-cyclohexylphenyl)-3-methoxypropionate; m.p. 249.5 - 251°C. (decomposed).

Analysis:

| | | | | |
|---|---|---|---|---|
| calc. for $C_{16}H_{20}O_3ClNa$ | : | C 60.28%; | H 6.32%; | Cl 11.12% |
| found | : | 59.02%; | 6.28%; | 10.38% |

I.R. $\nu_{max}^{Nujol}$ : 1600 $cm^{-1}$

N.M.R. $\delta_{ppm}^{D_2O}$ : 0.89 to 2.13 (10H, m)

2.64 to 3.10 (1H, m)
3.25 (3H, s)
3.36 to 4.17 (3H, m)
7.10 to 7.56 (3H, m)

Example 5

(1) A solution of 7.595 g. methyl 2-(3-chloro-4-cyclohexylphenyl) acetate and 2.052 g. methyl formate in 5 ml. anhydrous diethyl ether was added dropwise to a suspension of 0.821 g. sodium hydride, which had been prepared by washing 50% sodium hydride (1.642 g.) with petroleum ether in 40 ml. anhydrous diethyl ether for 20 minutes at 25 - 30°C., with stirring, and the reaction mixture was stirred at the same temperature for 3 hours. The reaction mixture was then poured into ice-water and the aqueous layer was separated off and washed with diethyl ether. The aqueous solution was mixed with diethyl ether and adjusted to pH 1 with 10% hydrochloric acid, while cooling. The ethereal layer was separated, washed with water, dried over anhydrous magnesium sulphate and then evaporated to dryness under reduced pressure to give colourless crystals. These crystals were dissolved in hot benzene and the solution was evaporated under reduced pressure. These operations were repeated three times and the resultant residue was finally treated with n-hexane. The insoluble substance was filtered off to give 228 mg. of colourless crystals of methyl 2-(3-chloro-4-cyclohexylphenyl)-3-hydroxyacrylate.

I.R. $\nu_{max}^{Nujol}$ : 3150, 1630 $cm^{-1}$

The n-hexane filtrate was evaporated to dryness under reduced pressure to give 5.14 g. of oily methyl 2-formyl-2-(3-chloro-4-cyclohexylphenyl) acetate.

I.R. $\nu_{max}^{liq.film}$: 1720, 1660, 1600 $cm^{-1}$

(2) 4.8 g. of the methyl 2-formyl-2-(3-chloro-4-cyclohexylphenyl) acetate obtained were added to a mixture of 5 ml. tetrahydrofuran and 10 ml. of a saturated aqueous solution of carbonic acid and the reaction mixture was stirred, with ice-cooling, for 10 minutes. 1.716 g. sodium borohydride were added all at once to the solution and the mixture was stirred, while cooling with ice, for 5 minutes. Diethyl ether was added to the reaction mixture, followed by treatment with 10% hydrochloric acid to decompose excess sodium borohydride. The organic layer was separated off and the remaining aqueous layer was extracted with diethyl ether. The extract was combined with the organic layer obtained above, washed with a saturated aqueous solution of sodium chloride, dried over anhydrous magnesium sulphate and then evaporated to dryness. The oily residue was dissolved in a small amount of chloroform, chromatographed on a silica gel column and then eluted with chloroform to give 3.59 g. of oily methyl _m_-chloro-_p_-cyclohexyl tropate.

I.R. $\nu_{max}^{liq.\ film}$ : 3420, 1730 $cm^{-1}$

N.M.R. $\delta_{ppm}^{CDCl_3}$ : 1.08 to 2.08 (10H, m)
2.52 (1H, t, J=6Hz)
2.80 to 3.14 (1H, m)
3.70 (3H, s)
3.68 to 4.28 (3H, m)
7.06 to 7.36 (3H, m)

Final elution with a mixture of chloroform and acetone gave 0.637 g. of crystalline 2-(3-chloro-4-cyclohexylphenyl)propane-1,3-diol as a by-product, which was identical with the compound prepared in Example 7-(1).

(3) 1.76 g. Acetic anhydride was added dropwise to a solution of 3.41 g. methyl _m_-chloro-_p_-cyclohexyl tropate in 4 ml. pyridine over the course of 10 minutes,

while stirring, and the reaction mixture was then stirred, with ice-cooling, for 2 hours. Diethyl ether was added to the reaction mixture, while cooling with ice and stirring, and the mixture then acidified with 10% hydrochloric acid. The organic layer was separated off and the remaining aqueous layer was extracted with diethyl ether. The extract was combined with the organic layer obtained above, washed successively with 10% hydrochloric acid, water, an aqueous solution of sodium bicarbonate and water, dried over anhydrous magnesium sulphate and then evaporated to dryness under reduced pressure. The oily residue was distilled under reduced pressure to give 2.904 g. methyl 3-acetoxy-2-(3-chloro-4-cyclohexylphenyl) propionate; b.p. 180 - 183°C./6 mm.Hg.

Analysis:

| | | | | |
|---|---|---|---|---|
| calc. for $C_{18}H_{23}O_4Cl$: | C 63.81%; | H 6.84%, | Cl 10.46% |
| found : | 63.93%; | 6.91%; | 10.59% |

I.R. $\nu_{max.}^{liq.\ film}$ : 1740 $cm^{-1}$

N.M.R. $\delta_{ppm}^{CCl_4}$ : 1.10 to 2.17 (10H, m)
1.97 (3H, s)
2.89 (1H, broad s)
3.68 (3H, s)
3.73 to 4.63 (3H, m)
7.07 to 7.36 (3H, m)

Example 6

(1) 9.535 g. Tosyl chloride were added portionwise to a solution of 18.11 g. methyl m-chloro-p-cyclohexyl tropate, prepared as in Examples 5-(1) and (2), in 50 ml. pyridine while stirring and cooling with ice, and the reaction mixture was then stirred at the same temperature for 1.5 hours. Ethyl acetate and ice-water were added to the reaction mixture and shaken. The organic layer was separated, washed successively

with cold 10% hydrochloric acid, water, a saturated aqueous solution of sodium bicarbonate and water, dried over anhydrous magnesium sulphate and then evaporated to dryness under reduced pressure to give 22.78 g. of oily methyl 3-tosyloxy-2-(3-chloro-4-cyclohexylphenyl) propionate which was used for the following reaction without further purification.

(2) A solution of 3.335 g. sodium in 60 ml. methanol was added dropwise to a solution of 22.77 g. methyl 3-tosyloxy-2-(3-chloro-4-cyclohexylphenyl) propionate in 250 ml. anhydrous methanol over the course of 45 minutes, while stirring at ambient temperature. After stirring the reaction mixture at ambient temperature for 5 hours, 7 ml. acetic acid were added dropwise to the reaction mixture. The mixture was evaporated to dryness under reduced pressure and ethyl acetate and ice-water were added to the resultant residue and shaken. The organic layer was separated off and the remaining aqueous layer was extracted with ethyl acetate. The extract was combined with the organic layer obtained above, washed successively with water, a saturated aqueous solution of sodium bicarbonate and water, dried over anhydrous magnesium sulphate and then evaporated to dryness under reduced pressure. The oily residue was chromatographed on a silica gel column and eluted with benzene to give 8.78 g. of oily methyl 2-(3-chloro-4-cyclohexylphenyl) 3-methoxypropionate which was identical with the compound obtained in the Example 4-(2).

Example 7

(1) 2.923 g. Sodium borohydride were added portionwise, over the course of 3 minutes, to a suspension of 7.952 g. methyl 2-formyl-2-(3-chloro-4-cyclohexylphenyl) acetate, prepared as in Example 5-(1), in a mixture of 9 ml. tetrahydrofuran and 16 ml. of a saturated aqueous solution of carbonic acid, and the

reaction mixture was stirred for 15 minutes, with ice-cooling. 8 ml. Tetrahydrofuran were added to the mixture, followed by stirring for 45 minutes, with ice-cooling. A further 8 ml. tetrahydrofuran were added to the mixture, which was then stirred at ambient temperature for 3 hours and adjusted to pH 1 with 10% hydrochloric acid, with ice-cooling.

The tetrahydrofuran was distilled off under reduced pressure and the residue was extracted with ethyl acetate. The extract was washed successively with an aqueous solution of sodium chloride, a saturated aqueous solution of sodium bicarbonate, water and an aqueous solution of sodium chloride, dried over anhydrous magnesium sulphate and then concentrated under reduced pressure. The precipitate in the concentrate was dissolved by heating and the solution was then left to stand at ambient temperature. The precipitate was filtered off with suction, washed with cold ethyl acetate and then dried to give 5.34 g. 2-(3-chloro-4-cyclohexylphenyl)propane-1,3-diol. An additional crop of 0.975 g. was recovered from the mother liquor. Total yied 6.315 g.; m.p. 123.5 - 124.5°C.

Analysis:

| | | | | |
|---|---|---|---|---|
| calc. for $C_{15}H_{21}O_2Cl$ : | C 67.03%; | H 7.88%; | Cl 13.19% |
| found : | 67.03%; | 7.92%; | 13.17% |

I.R. $\nu_{max}^{Nujol}$ : 3320 $cm^{-1}$

N.M.R. $ppm^{DMSO-d_6}$ : 1.10 to 2.10 (10H, m)
2.59 to 3.13 (2H, m)
3.69 (4H, t, J=5Hz)
4.63 (2H, t, J=5Hz)
7.30 (3H, broad s)

(2) A solution of 15.573 g. 2-(3-chloro-4-cyclohexylphenyl)propane-1,3-diol in 55 ml. dimethyl formamide was added dropwise to a suspension of 1.46 g. sodium hydride, which had been prepared from 50% sodium

hydride (2.923 g.) by washing with petroleum ether, in 30 ml. dimethyl formamide, over the course of one minute, with stirring and ice-cooling, and the reaction mixture then stirred at the same temperature for 30 minutes. A solution of 8.648 g. methyl iodide in 15 ml. dimethyl formamide was added to the mixture over the course of 1.25 hours, with ice-cooling, and the mixture thereafter stirred at the same temperature for 2 hours, with cooling with ice-water for an hour and again at ambient temperature for 2 hours. The reaction mixture was poured into water and the mixture extracted with ethyl acetate. The extract was washed thoroughly with water, dried over anhydrous magnesium sulphate and then evaporated to dryness under reduced pressure. The oily residue was chromatographed on a column of 150 g. silica gel, eluting with chloroform and then with a mixture of chloroform and acetone. From the chloroform eluate there were obtained 3.02 g. 1,3-dimethoxy-2-(3-chloro-4-cyclohexylphenyl)propane as a by-product. This compound was distilled under reduced pressure to give 1.87 g. of the pure substance; b.p. 144-148°C./3 mm.Hg.

Analysis:

| | | | | |
|---|---|---|---|---|
| calc. for $C_{17}H_{25}O_2Cl$ : | C 68.79%; | H 8.49%; | Cl 11.94% |
| found : | 68.76%; | 8.62%; | 12.38% |

I.R. $\nu_{max}^{liq.\ film}$ : 1110 $cm^{-1}$

N.M.R. $\delta_{ppm}^{CDCl_3}$ : 1.12 to 2.00 (10H, m)
2.76 to 3.20 (2H, m)
3.26 (6H, s)
3.44 to 3.72 (4H, m)
7.01 to 7.22 (3H, m)

Furthermore, 9.994 g. 2-(3-chloro-4-cyclohexylphenyl)-3-methoxypropanol were obtained from the fractions eluted with a mixture of chloroform and acetone; b.p. 156 - 158°C/3mm.Hg.

<u>Analysis</u>:

calc. for $C_{16}H_{23}O_2Cl$ : C 67.95%; H 8.20%; Cl 12.54%

found : 68.08%; 8.42%; 12.69%

I.R. $\nu_{max}^{liq.\ film}$ : 3400 $cm^{-1}$

N.M.R. $\delta_{ppm}^{CDCl_3}$ : 1.13 to 2.10 (10H, m)
2.40 (1H, t, J=6Hz)
2.76 to 3.36 (2H, m)
3.36 (3H, s)
3.53 to 4.07 (4H, m)
7.10 to 7.36 (3H, m)

(3) A solution of 4.52 g. 2-(3-chloro-4-cyclohexylphenyl)-3-methoxypropanol in 15 ml. acetic acid was added dropwise to a solution of 4 g. chromium oxide in a mixture of 30 ml. acetic acid and 5 ml. water, with stirring and cooling below 5°C, and the reaction mixture then stirred at the same temperature for 3 hours and subsequently left to stand at ambient temperature for one day. Water and diethyl ether were added to the reaction mixture. After shaking, the ether layer was separated and the remaining aqueous layer was extracted with diethyl ether. The extract was combined with the ether layer obtained above, washed with water and then extracted with a saturated aqueous solution of sodium bicarbonate. The extract was washed with diethyl ether, adjusted to pH 1 with 10% hydrochloric acid, with stirring and ice-cooling, and then extracted with diethyl ether. The ether extract was washed with an aqueous solution of sodium chloride, dried over anhydrous magnesium sulphate and evaporated under reduced pressure. The oily residue was dissolved in benzene and the solution was evaporated to remove the remaining acetic acid azeotropically. The resultant residue was chromatographed on a silica gel column, eluting with chloroform, to give 1.24 g. of oily 2-(3-chloro-4-cyclohexylphenyl)-3-methoxypropionic acid. This compound gradually crystallized when left to stand at ambient temperature.

Analysis:

| | | | | |
|---|---|---|---|---|
| calc. for $C_{16}H_{21}O_3Cl$ | : | C 64.75%; | H 7.13%; | Cl 11.95% |
| found | : | 64.47%; | 7.30%; | 11.69% |

I.R. $\nu_{max}^{CHCl_3}$ : 1705 $cm^{-1}$

N.M.R. $\delta_{ppm}^{CDCl_3}$ : 1.10 to 2.17 (10H, m)
2.76 to 3.23 (1H, broad s)
3.37 (3H, s)
3.53 to 4.03 (3H, m)
7.17 to 7.46 (3H, m)
10.73 (1H, broad s)

Example 8

2.938 g. Acetic anhydride were added dropwise, over the course of 15 minutes, to a solution of 3.222 g. 2-(3-chloro-4-cyclohexylphenyl)propane-1,3-diol in 6 ml. pyridine, with stirring and ice-cooling, and the reaction mixture then stirred at the same temperature for 2 hours and at ambient temperature for a further 2 hours and subsequently left to stand overnight at ambient temperature. Pyridine was distilled off under reduced pressure and the oily residue was dissolved in ethyl acetate. The solution was washed successively with water, 10% hydrochloric acid, water, a saturated aqueous solution of sodium bicarbonate and water, dried over anhydrous magnesium sulphate and then evaporated under reduced pressure. The residue was distilled under reduced pressure to give 3.685 g. 1,3-diacetoxy-2-(3-chloro-4-cyclohexylphenyl)propane ; b.p. 191 - 193°C./3 mm.Hg. This compound crystallized upon standing at ambient temperature for a few days.

Analysis:

calc. for $C_{19}H_{25}O_4Cl$ : C 64.67%; H 7.14%; Cl 10.05%

found : 64.48%; 7.10%; 10.12%

I.R. $\nu_{max}^{liq.\ film}$ : 1730 $cm^{-1}$

N.M.R. $\delta_{ppm}^{CCl_4}$ : 1.06 to 2.05 (10H, m)
2.00 (6H, s)
2.76 to 3.10 (2H, m)
4.23 (4H, d, J=6Hz)
7.03 to 7.30 (3H, m)

Example 9

A solution of 16 g. methyl m-chloro-p-cyclohexyl tropate in 194.8 ml. tetrahydrofuran was added dropwise to a solution of 17.15 g. barium hydroxide in 198.4 ml. water, with ice-cooling, and the reaction mixture stirred at the same temperature for 2 hours and then at ambient temperature for 16 hours. The precipitate was filtered off and washed with tetrahydrofuran. Water and diethyl ether were added to the solid and the mixture was adjusted to pH 1 with hydrochloric acid. The ether layer was separated off, washed with water, dried over anhydrous magnesium sulphate and then evaporated to dryness under reduced pressure. The resultant crystalline residue was recrystallized from a mixture of benzene and ethyl acetate to give 11 g. m-chloro-p-cyclohexyl tropic acid; m.p. 164 - 166°C.

Analysis:

calc. for $C_{15}H_{19}O_3Cl$ : C 63.71%; H 6.77%; Cl 12.54%
found : 63.91%; 6.91%; 12.27%

I.R. $\nu_{max}^{Nujol}$ : 3400, 1695 $cm^{-1}$

N.M.R. $\delta_{ppm}^{DMSO-d_6}$ : 1.05 to 2.08 (10H, m)
2.72 to 3.10 (1H, m)
3.44 to 4.08 (3H, m)
4.60 to 5.40 (1H, broad s)
7.05 to 7.52 (3H, m)

Example 10

3.143 g. Tosyl chloride were added all at once to a solution of 4.238 g. 2-(3-chloro-4-cyclohexylphenyl)-3-methoxypropanol in 15 ml. pyridine with stirring and ice-cooling, and the reaction mixture was stirred at the same temperature for 3 hours and then at ambient temperature for one hour. Diethyl ether and water were added to the reaction mixture. After shaking, the ether layer was separated off, washed successively with 10% hydrochloric acid, an aqueous solution of sodium chloride, a saturated aqueous solution of sodium bicarbonate and an aqueous solution of sodium chloride, dried over anhydrous magnesium sulphate and then evaporated under reduced pressure to give 6.0 g. oily 1-methoxy-2-(3-chloro-4-cyclohexylphenyl)-3-tosyloxypropane.

I. R. $\nu_{max}^{liq. film}$ : 1360, 1170 $cm^{-1}$

N.M.R. $\delta_{ppm}^{CDCl_3}$ : 1.10 to 2.10 (10H, m)
2.43 (3H, s)
2.76 to 3.14 (2H, m)
3.25 (3H, s)
3.55 (2H, d, J=6Hz)
4.23 (2H, d,d, J=2Hz, 7Hz)
6.89 to 7.79 (7H, m)

Example 11

492 mg. Sodium borohydride were added portionwise, over the course of 2 minutes, to a solution of 3.829 g. methyl 2-formyl-2-(3-chloro-4-cyclohexylphenyl)-acetate and 1.16 g. piperidine in 40 ml. tetrahydrofuran, with stirring and ice-cooling, and the reaction mixture was stirred for half an hour with ice-cooling and at ambient temperature for 4 hours and then left to stand overnight at ambient temperature. Excess

sodium borohydride was decomposed with 10% hydrochloric acid and the mixture was evaporated under reduced pressure. Ethyl acetate and a saturated aqueous solution of sodium bicarbonate were added to the resultant residue. After shaking, the organic layer was separated off, washed with water, dried over anhydrous magnesium sulphate and then evaporated to dryness under reduced pressure. The residue was dissolved in a mixture of diethyl ether and water and the solution treated with 10% hydrochloric acid, with stirring. The resultant precipitate was filtered off with suction, washed with water and diethyl ether and then recrystallized from aqueous methanol containing a small amount of 10% hydrochloric acid to give 1.641 g. methyl 2-(3-chloro-4-cyclohexylphenyl)-3-piperidinopropionate hydrochloride; m.p. 198.5 - 200°C. (dec.)

Analysis:

calc. for $C_{21}H_{30}NO_2Cl.HCl$:

| | C | H | N | Cl |
|---|---|---|---|---|
| calc. | 62.99%; | 7.80%; | 3.50%; | 17.71% |
| found: | 63.02%; | 7.87%; | 3.67%; | 17.48% |

I.R. $\nu_{max}^{Nujol}$ : 2600 to 2300, 1755 $cm^{-1}$

N.M.R. $\delta_{ppm}^{CDCl_3}$ : 1.13 to 2.26 (16H, m)
2.30 to 4.03 (6H, m)
3.72 (3H, s)
4.76 (1H, d,d, J=4Hz, 9Hz)
7.13 to 7.46 (3H, m)

Example 12

(1) A solution of 2.76 g. sodium in 80 ml. n-propanol was added dropwise over the course of half an hour to a suspension of 12.48 g. methyl 2-(4-cyclohexylphenyl)-3-tosyloxypropionate in 80 ml. n-propanol, while stirring at ambient temperature, and the reaction mixture was then stirred at the same temperature for

one day. 7.2 ml. Acetic acid were added dropwise to the mixture and n-propanol was distilled off under reduced pressure from the reaction mixture. Ethyl acetate and water were added to the resultant residue. After shaking, the organic layer was separated off and the remaining aqueous layer was extracted with ethyl acetate. The extract and the organic layer obtained above were combined, washed successively with water, an aqueous solution of sodium bicarbonate and water, dried over anhydrous magnesium sulphate and then evaporated. The oily residue was dissolved in a small amount of benzene, chromatographed on a silica gel column and eluted with benzene to give 5.777 g. of oily propyl 2-(4-cyclohexylphenyl)-3-propoxypropionate.

I.R. $\nu_{max}^{liq.\ film}$ : 1730 $cm^{-1}$

N.M.R. $\delta_{ppm}^{CDCl_3}$ : 0.84 (6H, t, J=8Hz)
1.06 to 2.12 (14H, m)
2.28 to 2.68 (1H, m)
3.39 (2H, t, J=8Hz)
3.52 to 4.24 (5H, m)
7.04 to 7.36 (4H, m)

(2) A mixture of 14.044 g. propyl 2-(4-cyclohexylphenyl)-3-propoxypropionate, 4.061 g. methanesulphonic acid, 39 ml. 98% formic acid and 4 ml. water was heated under reflux for 2.5 hours while stirring. Formic acid was distilled off from the reaction mixture under reduced pressure and ethyl acetate and cold water added to the resultant residue. After shaking, the ethyl acetate layer was separated off, washed with water, dried over anhydrous magnesium sulphate and then evaporated to dryness under reduced pressure. The residue was dissolved in 30 ml. n-propanol and the solution was treated with a solution of 1.6 g. sodium hydroxide in 8 ml. water, with stirring

and ice-cooling, and then evaporated under reduced pressure. The residue was triturated with acetone and the resultant precipitate was filtered off, washed with acetone and then recrystallized from a mixture of n-propanol and acetone to give 10.9 g. sodium 2-(4-cyclohexylphenyl)-3-propoxypropionate; m.p. 223 - 224.5°C. This compound was identical to the specimen prepared in the following Example 13.

Example 13

16.64 g. Methyl 2-(4-cyclohexylphenyl)-3-tosyloxy-propionate were added all at once to a solution of 3.68 g. sodium in 200 ml. n-propanol, with stirring and ice-cooling, and the reaction mixture was stirred at the same temperature for half an hour and then at ambient temperature for 20 hours. The mixture was treated with 7.2 ml. acetic acid and evaporated to dryness under reduced pressure. Ice-water and diethyl ether were added to the residue. After shaking, the organic layer was separated off and the remaining aqueous layer was extracted with diethyl ether. The extract and the organic layer obtained above were combined and extracted with an aqueous solution of sodium bicarbonate. The extract was washed with diethyl ether, adjusted to pH 1 with 10% hydrochloric acid, with ice-cooling, and then extracted again with diethyl ether. The ether extract was dried over anhydrous magnesium sulphate and evaporated to dryness. The residue was treated with n-hexane and left to stand in a refrigerator. The precipitated 2-(4-cyclohexylphenyl)-acrylic acid (0.821 g.) was filtered off and the filtrate was evaporated under reduced pressure. The oily residue was dissolved in 20 ml. n-propanol, treated with a solution of 720 mg. sodium hydroxide in a small amount of water and then evaporated under reduced pressure. The residue was crystallized by triturating with acetone and the resultant precipitate

was filtered off with suction and recrystallized from a mixture of n-propanol and acetone to give 2.05 g. sodium 2-(4-cyclohexylphenyl)-3-propoxy-propionate; m.p. 223 - 224.5°C.

Analysis:

| | | | |
|---|---|---|---|
| calc. for $C_{18}H_{25}O_3Na$ : | C 69.21%; | H 8.07% |
| found : | 69.06%; | 8.09% |

I.R. $\nu_{max}^{Nujol}$ : 1585 $cm^{-1}$

N.M.R. $\delta_{ppm}^{D_2O}$ : 0.69 (3H, t, J=6Hz)
0.89 to 2.03 (12H, m)
2.03 to 2.59 (1H, broad s)
3.33 (2H, t, J=6Hz)
3.17 to 4.20 (3H, m)
7.07 (2H, d, J=8Hz)
7.31 (2H, d, J=8Hz)

Example 14

(1) Gaseous ammonia was introduced vigorously into a solution of 12.48 g. methyl 2-(4-cyclohexylphenyl)-3-tosyloxypropionate in 400 ml. methanol for 30 minutes, with stirring and ice-cooling, and stirring was continued at ambient temperature for 7.5 hours, while slowly bubbling gaseous ammonia through the solution. The reaction vessel was sealed and left to stand overnight in a refrigerator and then the solvent was distilled off under reduced pressure. The residue was treated with a mixture of diethyl ether and 5% hydrochloric acid and the resultant precipitate was filtered off, washed with a mixture of diethyl ether and water and then treated with a mixture of diethyl ether and an aqueous solution of sodium bicarbonate to give oily methyl 2-(4-cyclohexylphenyl)-3-aminopropionate. The aqueous layer was separated from the filtrate, washed with diethyl ether, rendered alkaline with sodium bicarbonate, while cooling with ice, and then

extracted with diethyl ether. The extract was washed with water, dried over anhydrous magnesium sulphate and evaporated under reduced pressure to give the same oily product. Total yield 5.67 g.

I.R. $\nu_{max}^{liq.\ film}$ : 3350, 3300 $cm^{-1}$

N.M.R. $\delta_{ppm}^{CDCl_3}$ : 1.08 to 2.04 (10H, m)
2.28 to 2.64 (1H, m)
2.78 to 3.42 (2H, m)
3.50 to 3.72 (1H, m)
3.64 (3H, s)
7.16 (4H, s)

(2) A solution of 3.779 g. mesyl chloride in 15 ml. methylene chloride was added dropwise over the course of 40 minutes to a mixture of 8.613 g. methyl 2-(4-cyclohexylphenyl)-3-aminopropionate, 4 g. triethylamine, 3.326 g. sodium bicarbonate and 50 ml. methylene chloride, with stirring and ice-cooling, and the reaction mixture was stirred at the same temperature for half an hour. The reaction mixture was evaporated under reduced pressure and a mixture of ethyl acetate and water was added to the residue. After shaking, the organic layer was separated and the remaining aqueous layer was extracted with ethyl acetate. The extract and the organic layer were combined, washed successively with water, 5% hydrochloric acid and water, dried over anhydrous magnesium sulphate and then concentrated under reduced pressure. The precipitate in the concentrate was dissolved again by heating and the solution was cooled to ambient temperature. The precipitate was filtered off with suction to give 2.802 g. crystalline methyl 2-(4-cyclohexylphenyl)-3-mesylaminopropionate. 2.058 g. of the same product were recovered by concentration and cooling. Total yield 4.860 g.; m.p. 138 - 140°C.

I. R. $\nu_{max}^{Nujol}$ : 3430, 3150, 1300, 1140 $cm^{-1}$

N.M.R. $\delta_{ppm}^{DMSO-d_6}$ : 1.08 to 1.96 (10H, m)
2.32 to 2.56 (1H, m)
2.80 (3H, s)
3.17 (1H, d,d, J=8Hz, 12Hz)
3.32 (1H, s)
3.42 to 3.92 (2H, m)
3.59 (3H, s)
7.19 (4H, s)

(3) A solution of 4.068 g. methyl 2-(4-cyclohexylphenyl)-3-mesylaminopropionate in 60 ml. tetrahydrofuran was added dropwise, over the course of an hour, to a suspension of 910.8 mg. lithium aluminium hydride in 40 ml. tetrahydrofuran, with stirring and ice-cooling, and the reaction mixture was stirred at the same temperature for half an hour. Ethyl acetate was added dropwise to the reaction mixture to decompose excess lithium aluminium hydride and then the reaction mixture was evaporated under reduced pressure. A mixture of ethyl acetate and 5% hydrochloric acid was added to the residue. The organic layer was separated off and the remaining aqueous layer was extracted with ethyl acetate. The extract and the organic layer were combined, washed with 5% hydrochloric acid and then with water, dried over anhydrous magnesium sulphate and then evaporated to dryness under reduced pressure. The resultant crystals were recrystallized from benzene to give 3.104 g. 2-(4-cyclohexylphenyl)-3-mesylaminopropanol; m.p. 99 - 100.5°C.

<u>Analysis:</u>

calc. for $C_{16}H_{25}NO_3S$ :

| | C | H | N | S |
|---|---|---|---|---|
| calc. | 61.70%; | 8.09%; | 4.50%; | 10.30% |
| found: | 61.66%; | 8.15%; | 4.45%; | 10.14% |

I.R. $\nu_{max}^{Nujol}$ : 3500, 3200, 1300, 1140 $cm^{-1}$

N.M R. $\delta_{ppm}^{DMSO-d_6}$ : 1.05 to 2.05 (10H, m)
2.25 to 3.03 (2H, m)
2.74 (3H, s)
3.18 (2H, m)
3.58 (2H, t, J=5Hz)
4.64 (1H, t, J=5Hz)
6.81 (1H, t, J=6Hz)
7.12 (4H, s)

Example 15

(1) 24.96 g. Methyl 2-(4-cyclohexylphenyl)-3-tosyloxypropionate and 4.95 g. nitroethane were dissolved in 140 ml. hot methanol and the solution left to stand at ambient temperature. Over the course of 35 minutes, a solution of 2.898 g. sodium in 35 ml. methanol was added dropwise, with stirring at ambient temperature to the solution and the mixture stirred at the same temperature for 6 hours. Over the course of 20 minutes, a solution of 1.38 g. sodium in 50 ml. methanol was added dropwise with stirring and ice-cooling to the reaction mixture. The mixture was then added dropwise, over the course of an hour, to a mixture of 80 ml. 10% hydrochloric acid and 250 ml. methanol with stirring and ice-cooling, and the mixture was stirred at the same temperature for an hour and then at ambient temperature overnight. Methanol was distilled off under reduced pressure and the residue was extracted with ethyl acetate. The extract was washed successively with water, a saturated aqueous solution of sodium bicarbonate and water, dried over anhydrous magnesium sulphate and then evaporated under reduced pressure. The resultant crystals were recrystallized from methanol to give 5.742 g. methyl 2-(4-cyclohexylphenyl)-3-acetylpropionate; m.p. 86.5 - 90°C.

I.R. $\nu_{max}^{liq.\ film}$ : 1740 (sh), 1720 $cm^{-1}$

N.M.R. $\delta_{ppm}^{CDCl_3}$ : 1.07 to 2.07 (10H, m)
2.15 (3H, s)
2.30 to 2.59 (1H, m)
2.66 (1H, d,d, J=4Hz, 18Hz)
3.25 (1H, d,d, J=10Hz, 18Hz)
3.63 (3H, s)
4.07 (1H, d,d, J=4Hz, 10Hz)
7.13 (4H, s)

(2) A mixture of 2.448 g. methyl 2-(4-cyclohexylphenyl)-3-acetylpropionate, 50 ml. methanol and 6.8 ml. 10% aqueous sodium hydroxide solution was heated under reflux, with stirring, for 10 minutes. Methanol was then distilled off under reduced pressure and the precipitated crystals were filtered off with suction and washed with water and then with ethyl acetate. These crystals were dissolved in a mixture of ethyl acetate and water and the solution adjusted to pH 1 with 10% hydrochloric acid, with ice-cooling. The ethyl acetate layer was separated, washed with water, dried over anhydrous magnesium sulphate and then evaporated to dryness under reduced pressure. The resultant crystals were recrystallized from a mixture of benzene and n-hexane to give 1.972 g. 2-(4-cyclohexylphenyl)-3-acetylpropionic acid; m.p. 113.5 - 115°C.

Analysis:

| | | | |
|---|---|---|---|
| calc. for $C_{17}H_{22}O_3$ | : | C 74.42%, | H 8.08% |
| found | : | 74.33%; | 8.11% |

I.R. $\nu_{max}^{Nujol}$ : 1720, 1700 $cm^{-1}$

N.M.R. $\delta_{ppm}^{CDCl_3}$ : 1.10 to 2.05 (10H, m)
2.13 (3H, s)
2.20 to 2.50 (1H, m)

2.65 (1H, d,d, J=5Hz, 18Hz)
3.33 (1H, d,d, J=10Hz, 18Hz)
4.07 (1H, d,d, J=5Hz, 10Hz)
7.08 to 7.53 (4H, m)
9.46 to 10.00 (1H, broad s)

Example 16

(1) A mixture of 3.744 g. methyl 2-(4-cyclohexyl phenyl)-3-acetylpropionate, 1 ml. ethylene glycol, 10 mg. p-toluenesulphonic acid monohydrate and 10 ml. benzene was heated under reflux, with stirring, for 5 hours, while removing water azetropically. After cooling, the reaction mixture was shaken with a mixture of benzene and an aqueous solution of sodium bicarbonate The organic layer was separated, washed with water, dried over anhydrous magnesium sulphate and then evaporated to dryness to give 4.356 g. of oily methyl 2-(4-cyclohexylphenyl)-4,4-ethylenedioxyvalerate.

I. R. $\nu_{max}^{liq.\ film}$ : 1735, 1140, 1040 $cm^{-1}$

N.M.R. $\delta_{ppm}^{CDCl_3}$ : 1.04 to 2.12 (11H, m)
1.31 (3H, s)
2.28 to 2.60 (1H, m)
2.74 (1H, d,d, J=10Hz, 14Hz)
3.61 (3H, s)
3.72 to 3.88 (1H, m)
3.91 (4H, s)
7.04 to 7.40 (4H, m)

(2) A solution of 4.648 g. methyl 2-(4-cyclohexylphenyl)-4,4-ethylenedioxyvalerate in 25 ml. diethyl ether was added dropwise over the course of 20 minutes to a suspension of 531.3 mg. lithium aluminum hydride in 50 ml. diethyl ether, with stirring and ice-cooling, and the mixture then stirred at the same temperature for half an hour. After decomposing excess lithium aluminium hydride by adding dropwise 3 ml.

ethyl acetate, 10 ml. of a 15% aqueous solution of sodium hydroxide were added dropwise to the reaction mixture, with stirring and ice-cooling, whereafter a mixture of diethyl ether and a 15% aqueous solution of sodium hydroxide was added to the mixture. After shaking, the organic layer was separated and the remaining aqueous layer was extracted with diethyl ether. The extract and the organic layer obtained above were combined, washed with an aqueous solution of sodium chloride, dried over anhydrous magnesium sulphate and then evaporated under reduced pressure. The oily residue was dried in a desiccator under reduced pressure to give 4.15 g. oily 2-(4-cyclohexylphenyl)-4,4-ethylenedioxypentanol.

I.R. $\nu_{max}^{liq.\ film}$ : 3450, 1140, 1040 $cm^{-1}$

N.M.R. $\delta_{ppm}^{CDCl_3}$ : 1.10 to 2.01 (10H, m)
1.30 (3H, s)
2.09 (2H, d, J=6Hz)
2.26 to 2.69 (2H, broad s)
3.00 (1H, quintet, J=6Hz)
3.56 to 3.86 (2H, m)
3.92 (4H, s)
7.10 (4H, s)

(3) A solution of 4,378 g. 2-(4-cyclohexylphenyl)-4,4-ethylenedioxypentanol in 44 ml. acetone was stirred with 2 ml. 10% hydrochloric acid at ambient temperature for half an hour. Acetone was distilled off under reduced pressure and the residue was shaken with a mixture of diethyl ether and an aqueous solution of sodium chloride. The organic layer was separated off and the remaining aqueous layer was extracted with diethyl ether. The extract and the organic layer obtained above were combined, washed with an aqueous solution of sodium chloride solution, dried over anhydrous magnesium

sulphate and then evaporated to dryness under reduced pressure. The oily residue was dissolved in a small amount of benzene, chromatographed on an alumina column and eluted successively with benzene, chloroform and then with a mixture of chloroform and acetone. The fractions obtained with chloroform and with a mixture of chloroform and acetone were combined, concentrated under reduced pressure, chromatographed on a silica gel column and eluted successively with benzene, a mixture of benzene and chloroform, chloroform and then with a mixture of chloroform and acetone. The fractions eluted with chloroform and a mixture of chloroform and acetone were combined and evaporated under reduced pressure. The residue was dried under reduced pressure (2 mm.Hg) for 2 hours to give 2.553 g. of oily 2-(4-cyclohexylphenyl)-3-acetylpropanol which was left to stand for a few days to crystallize.

Analysis:

| | | |
|---|---|---|
| calc. for $C_{17}H_{24}O_2$ | : | C 78.42%; H 9.29% |
| found | : | 78.74%; 9.48% |
| I.R. $\nu_{max}^{liq.\ film}$ | : | 3400, 1710 $cm^{-1}$ |
| N.M.R. $\delta_{ppm}^{CDCl_3}$ | : | 1.06 to 2.02 (10H, m) |
| | | 2.08 (3H, s) |
| | | 2.20 to 2.64 (3H, m) |
| | | 2.82 (1H, t, J=8Hz) |
| | | 3.34 (1H, t, J=8Hz) |
| | | 3.52 to 3.96 (2H, m) |
| | | 7.05 to 7.32 (4H, m) |

Example 17

(1) A solution of 8.625 g. sodium in 100 ml. anhydrous methanol was added dropwise to a solution of 52 g. methyl 2-(4-cyclohexylphenyl)-3-tosyloxypropionate in 300 ml. anhydrous methanol over the course of 35 minutes at ambient temperature and then the mixture was

stirred for 20 hours. The mixture was acidified with 22.7 g. acetic acid and evaporated under reduced pressure. The residue was shaken with a mixture of 150 ml. ethyl acetate and 150 ml. water and the ethyl acetate layer was separated off. The remaining aqueous layer was extracted twice with 50 ml. amounts of ethyl acetate and the extracts and the ethyl acetate layer obtained above were combined, washed successively with water, a saturated aqueous solution of sodium bicarbonate and water, dried over anhydrous magnesium sulphate and then evaporated under reduced pressure to give 35 g. methyl 2-(4-cyclohexylphenyl)-3-methoxypropionate.

I.R. $\nu_{max.}^{liq.\ film}$ : 1740 $cm^{-1}$

N.M.R. $\delta_{ppm}^{CDCl_3}$ : 1.10 to 2.10 (10H, m)
2.21 to 2.68 (1H, broad s)
3.33 (3H, s)
3.45 to 4.12 (3H, m)
3.64 (3H, s)
7.12 to 7.36 (4H, m)

(2) To a solution of 8.85 g. methyl 2-(4-cyclohexylphenyl)-3-methoxypropionate in 180 ml. methanol were added 22 ml. of a 10% aqueous solution of sodium hydroxide and the mixture was stirred at ambient temperature for 1.5 hours. Methanol was distilled off under reduced pressure and water was added to the residue. Insoluble material was filtered off and dissolved in a mixture of ethyl acetate and water. The solution was adjusted to pH 1 with 10% hydrochloric acid, while cooling with ice-water. The ethyl acetate layer was separated off and the remaining aqueous layer was extracted with ethyl acetate. The extract and the ethyl acetate layer were combined, washed with water, dried over anhydrous magnesium sulphate and then evaporated to dryness. The crystalline residue

was recrystallized from n-hexane to give 4.8 g. 2-(4-cyclohexylphenyl)-3-methoxypropionic acid; m.p. 84 - 85°C.

Analysis:

| | | | |
|---|---|---|---|
| calc. for $C_{16}H_{22}O_3$ | : | C 73.25%; | H 8.45% |
| found | : | 73.25%; | 8.63% |

I.R. $\nu_{max}^{Nujol}$ : 1710 $cm^{-1}$

N.M.R. $\delta_{ppm}^{CDCl_3}$ : 1.10 to 2.13 (10H, m)
2.26 to 2.79 (1H, m)
3.36 (3H, s)
3.50 to 4.20 (3H, m)
7.00 to 7.40 (4H, m)
11.43 (1H, s)

Example 18

(1) A solution of 11 g. methyl 2-(4-cyclohexylphenyl)-3-methoxypropionate in 40 ml. anhydrous diethyl ether was added dropwise over the course of 28 minutes to a suspension of 1.087 g. lithium aluminium hydride in 85 ml. anhydrous diethyl ether, with stirring and ice-cooling, and the mixture stirred at the same temperature for 40 minutes. To the mixture were added dropwise 25 ml. ethyl acetate and 10% hydrochloric acid, while cooling in an ice-water bath. The organic layer was separated off and the remaining aqueous layer was extracted with diethyl ether. The extract and the organic layer obtained above were combined, washed successively with a saturated aqueous solution of sodium bicarbonate and water, dried over anhydrous magnesium sulphate and then evaporated under reduced pressure. The oily residue was fractionated by vacuum distillation to give 7.87 g. 2-(4-cyclohexylphenyl)-3-methoxypropanol; b.p. 166 - 168°C./2 mm.Hg.

I.R. $\nu_{max}^{liq.\ film}$ : 3425, 1120 $cm^{-1}$

N.M.R. $\delta_{ppm}^{CDCl_3}$ : 1.05 to 2.10 (10H, m)
2.20 to 2.68 (1H, m)
2.52 (1H, s)
2.91 to 3.3 (1H, m)
3.31 (3H, s)
3.5 to 4.05 (4H, m)
6.87 to 7.28 (4H, m)

(2) 408 mg. Acetic anhydride were added to a solution of 496.7 mg. 2-(4-cyclohexylphenyl)-3-methoxypropanol in 2 ml. pyridine, while cooling in an ice-water bath, and the mixture was stirred at 5 - 8°C for 2 hours and at ambient temperature for an additional 5 hours and then left to stand in a refrigerator for 63 hours. The reaction mixture was diluted with ethyl acetate, washed successively with cold water, cold 10% hydrochloric acid, water, a saturated aqueous solution of sodium bicarbonate and water, dried over anhydrous magnesium sulphate and then evaporated under reduced pressure to give 500 mg. of colourless, oily 1-acetoxy-2-(4-cyclohexylphenyl)-3-methoxypropane.

I.R. $\nu_{max}^{liq.\ film}$ : 1740 $cm^{-1}$

N.M.R. $\delta_{ppm}^{CDCl_3}$ : 1.10 to 2.05 (10H, m)
1.95 (3H, s)
2.28 to 2.62 (1H, m)
3.04 to 3.32 (1H, m)
3.28 (3H, s)
3.60 (2H, d, J=7Hz)
4.32 (2H, d, J=7Hz)
7.12 (4H, broad s)

Example 19

337.3 mg. Benzoyl chloride were added dropwise to a solution of 496.7 mg. 2-(4-cyclohexylphenyl)-3-methoxypropanol in 2 ml. pyridine, while cooling in an ice-water bath, and the reaction mixture then stirred at the same temperature for half an hour. Ethyl acetate and

cold water were added to the reaction mixture. After shaking, the organic layer was separated off, washed successively with cold 10% hydrochloric acid, water, a saturated aqueous solution of sodium bicarbonate and water, dried over anhydrous magnesium sulphate and then evaporated under reduced pressure to give 700 mg. of colourless, oily 1-benzoyloxy-2-(4-cyclohexylphenyl)-3-methoxypropane.

I.R. $\nu_{max}^{liq. film}$ : 1725 $cm^{-1}$

N.M.R. $\delta_{ppm}^{CDCl_3}$ : 1.03 to 2.10 (10H, m)
2.23 to 2.63 (1H, m)
3.17 to 3.56 (1H, m)
3.31 (3H, s)
3.71 (2H, d, J=6Hz)
4.56 (2H, d, J=6Hz)
7.02 to 7.56 (7H, m)
7.86 to 8.13 (2H, m)

Example 20

A solution of 5.46 g. 4-chlorophenoxyacetyl chloride in 10 ml. methylene chloride was added dropwise, over the course of 10 minutes, to a solution of 6 g. 2-(4-cyclohexylphenyl)-3-methoxypropanol in 5.74 g. pyridine, while cooling in an ice-water bath, and the mixture was stirred at the same temperature for half an hour. Methylene chloride was distilled off under reduced pressure and ethyl acetate and cold water added to the residue. After shaking the mixture, the ethyl acetate layer was separated off, washed successively with cold 10% hydrochloric acid, a saturated aqueous solution of sodium bicarbonate and water, dried over anhydrous magnesium sulphate and then evaporated under reduced pressure to give 11 g. of yellowish, oily 1-(4-chlorophenoxyacetoxy)-2-(4-cyclohexylphenyl)-3-methoxypropane.

I.R. $\nu_{max}^{liq.\ film}$ : 1770, 1740 (sh) $cm^{-1}$

N.M.R. $\delta_{ppm}^{CDCl_3}$ : 1.08 to 2.12 (10H, m)
2.48 (1H, broad s)
3.10 to 3.36 (1H, m)
3.26 (3H, s)
3.53 (2H, d, J=6Hz)
4.36 to 4.68 (2H, m)
4.48 (2H, s)
6.71 (2H, d, J=8Hz)
7.04 to 7.32 (6H, m)

Example 21

(1) 7.76 g. of a 30% ethanolic solution of methylamine were added dropwise to a solution of 10.4 g. methyl 2-(4-cyclohexylphenyl)-3-tosyloxypropionate in 100 ml. methanol over the course of 5 minutes at ambient temperature and the mixture then stirred for 2.5 hours. Methanol was distilled off under reduced pressure. The residue was shaken with a mixture of diethyl ether, water and a saturated aqueous solution of sodium bicarbonate and the ether layer was separated off, washed with water and then extracted with 10% hydrochloric acid. The aqueous extract was washed with diethyl ether, neutralized with a saturated aqueous solution of sodium bicarbonate until it became turbid and extracted with diethyl ether. The extract was washed with water, dried over anhydrous magnesium sulphate and then evaporated to dryness under reduced pressure to give 6.7 g. methyl 2-(4-cyclohexylphenyl)-3-methylaminopropionate.

I.R. $\nu_{max}^{liq.\ film}$ : 3340, 1720 $cm^{-1}$

N.M.R. $\delta_{ppm}^{CDCl_3}$ : 1.14 to 2.04 (10H, m)
1.48 (1H, s)
2.40 (3H, s)
2.28 to 2.64 (1H, m)

2.84 (1H, d,d, J=7Hz, 12Hz)
3.22 (1H, d,d, J=8Hz, 12Hz)
3.65 (3H, s)
3.79 (1H, d,d, J=7Hz, 8Hz)
7.28 (4H, broad s)

(2) A solution of 6 g. methyl 2-(4-cyclohexylphenyl)-3-methylaminopropionate in 10 ml. anhydrous diethyl ether was added dropwise to a suspension of 827 mg. lithium aluminium hydride in 40 ml. anhydrous diethyl ether over the course of 25 minutes, while cooling in an ice-water bath, and the mixture then stirred at the same temperature for 3 hours. To the reaction mixture were added dropwise 15 ml. ethyl acetate and then a 15% aqueous solution of sodium hydroxide, whereafter the mixture was extracted with diethyl ether. The extract was washed with a saturated aqueous solution of sodium chloride, dried over anhydrous magnesium sulphate and then evaporated to dryness under reduced pressure. The residue was dissolved in hot ethanolic hydrogen chloride, and diethyl ether was added to the cooled solution. The mixture was left to stand over-night and the precipitated crystals were filtered off and washed with diethyl ether to give 1.5 g. 2-(4-cyclohexylphenyl)-3-methylaminopropanol hydrochloride. 500 mg. of the same hydrochloride were obtained from the mother liquor. These products were combined and recrystallized from isopropanol to give 1.8 g. of the pure hydrochloride; m.p. 178 - 180°C.

Analysis:

calc. for $C_{16}H_{25}NO \cdot HCl$ :

C 67.7%; H 9.23%; N 4.94%, Cl 12.49%

found : 67.50%; 9.30%; 4.93%; 12.74%

I.R. $\nu_{max}^{Nujol}$ : 3300 to 2400 $cm^{-1}$

N.M.R. $\delta_{ppm}^{D_2O}$ : 1.08 to 2.20 (10H, m)
2.30 to 2.76 (1H, m)

2.83 (3H, s)
3.23 to 4.07 (5H, m)
7.36 (4H, t, J=10Hz)

Example 22

A mixture of a solution of 3.5 g. methyl 2-(4-cyclohexylphenyl)-3-methylaminopropionate in 15 ml. isopropanol and a solution of 508.3 mg. sodium hydroxide in 1 ml. water was stirred at 70 - 80°C. for 2 hours and then left to stand at ambient temperature. The precipitated crystals were filtered off with suction, washed with a mixture of isopropanol and water (15:1 v/v) and then with isopropanol to give 2.2 g. sodium 2-(4-cyclohexylphenyl)-3-methylaminopropionate monohydrate; m.p. above 260°C.

Analysis:

calc. for $C_{16}H_{22}NO_2N_a \cdot H_2O$ :

C 63.47%; H 8.04%; N 4.63%

found : 63.20; 8.03%; 4.50%

I.R. $\nu_{max}^{Nujol}$ : 1590, 3310 $cm^{-1}$

N.M.R. $\delta_{ppm}^{D_2O}$ : 0.89 to 2.00 (10H, m)
2.20 (3H, s)
2.30 to 2.76 (2H, m)
3.06 (1H, t, J=8Hz)
3.61 (1H, t, J=8Hz)
7.03 (2H, d, J=8Hz)
7.26 (2H, d, J=8Hz)

Example 23

A solution of 1.242 g. methyl 2-(3-chloro-4-cyclohexylphenyl)-3-methoxypropionate in 10 ml. anhydrous diethyl ether was added dropwise over the course of 15 minutes to a suspension of 152 mg. lithium aluminium hydride in anhydrous diethyl ether, with stirring and ice-cooling, and the reaction mixture

then stirred at the same temperature for half an hour. After decomposing excess lithium aluminium hydride by adding ethyl acetate, with stirring and ice-cooling. the mixture was shaken with a mixture of water 10% hydrochloric acid and diethyl ether and the organic layer was separated off. The remaining aqueous layer was filtered and the filtrate was extracted with diethyl ether. The extract and the organic layer were combined, washed with an aqueous solution of sodium chloride, dried over anhydrous magnesium sulphate and then evaporated under reduced pressure to give 1.116 g. of oily 2-(3-chloro-4-cyclohexylphenyl)-3-methoxypropanol.

I.R. $\nu_{max}^{liq.\ film}$ : 3400 $cm^{-1}$

N.M.R. $\delta_{ppm}^{CDCl_3}$ : 1.10 to 2.07 (10H, m)
2.57 (1H, broad s)
2.74 to 3.23 (2H, m)
3.28 (3H, s)
3.56 to 3.91 (4H, m)
7.07 to 7.30 (3H, m)

Example 24

(1) A solution of 4.875 g. nitroethane in 15 ml. methanol was added dropwise over the course of 10 minutes to a solution of 2.243 g. sodium in 100 ml. methanol, while stirring at ambient temperature. To the mixture was added dropwise, over the course of an hour, a solution of 28.175 g. methyl 2-(3-chloro-4-cyclohexylphenyl)acrylate in 65 ml. methanol, while stirring at ambient temperature, and the mixture stirred at the same temperature for 3 hours. To the mixture was added dropwise an additional solution of 1.495 g. sodium in 40 ml. methanol over the course of 20 minutes, with stirring and ice-cooling. The reaction mixture was poured dropwise into a chilled mixture of 200 ml. of 10% hydrochloric acid and 250 ml. methanol,

with stirring. After stirring with ice-cooling for an hour and then at ambient temperature overnight, methanol was distilled off under reduced pressure. The aqueous solution was extracted with ethyl acetate and the extract was washed with water, dried over anhydrous magnesium sulphate and then evaporated under reduced pressure. The oily residue was dissolved in a small amount of benzene, chromatographed on a silica gel column, eluted with benzene and then with a gradient mixture of benzene and chloroform (10:1 - 1:10 v/v) to give 15.213 g. methyl 2-(3-chloro-4-cyclohexylphenyl)-3-acetylpropionate.

I.R. $\nu_{max}^{liq.\ film}$ : 1730, 1715 $cm^{-1}$

N.M.R. $\delta_{ppm}^{CDCl_3}$ : 1.07 to 2.10 (10H, m)
2.17 (3H, s)
2.66 (1H, d,d, J=5Hz, 18Hz)
3.02 (1H, broad s)
3.23 (1H, d,d, J=8Hz, 18Hz)
3.68 (3H, s)
4.07 (1H, d,d, J=5Hz, 8Hz)
7.13 to 7.43 (3H, m)

(2) 4.193 g. Methyl 2-(3-chloro-4-cyclohexylphenyl)-3-acetylpropionate were added to a solution of 0.78 g. sodium hydroxide in 7 ml. water and 35 ml. methanol and the mixture heated under reflux for 30 minutes, while stirring. Methanol was distilled off under reduced pressure. The residual mixture was adjusted to pH 1 with 10% hydrochloric acid and extracted with ethyl acetate. The extract was washed with water, dried over anhydrous magnesium sulphate and then evaporated under reduced pressure. The oily residue was triturated with a mixture of benzene and n-hexane and the precipitated crystals were filtered off with suction and washed with a mixture of benzene and n-hexane to give 2.763 g. 2-(3-chloro-4-cyclohexylphenyl)-3-acetylpropionic acid;

m.p. 117 - 118.5°C.

Analysis:

calc. for $C_{17}H_{21}O_3Cl$ : C 66.12%; H 6.85%, Cl 11.48%
found : 65.84%; 6.77%; 11.71%

I.R. $\nu_{max}^{Nujol}$ : 1720, 1710 (sh) $cm^{-1}$

N.M.R. $\delta_{ppm}^{CDCl_3}$ : 1.10 to 2.03 (10H, m)
2.13 (3H, s)
2.64 (1H, d,d, J=5Hz, 17Hz)
3.00 (1H, broad s)
3.31 (1H, d,d, J=10Hz, 17Hz)
4.05 (1H, d,d, J=5Hz, 10Hz)
7.13 to 7.38 (3H, m)

Example 25

(1) A mixture of 7.256 g. methyl 2-(3-chloro-4-cyclohexylphenyl)-3-acetylpropionate, 5 ml. ethylene glycol, 30 mg. p-toluenesulphonic acid monohydrate and 50 ml. benzene was heated under reflux for 6 hours, while stirring, the water being distilled off azeotropically. After cooling, the organic layer was separated off and the aqueous layer was extracted with benzene. The extract and the organic layer were combined, washed with water, dried over anhydrous magnesium sulphate and then evaporated to dryness, under reduced pressure, to give 7.576 g. methyl 2-(3-chloro-4-cyclohexylphenyl)-4,4-ethylenedioxyvalerate.

I. R. $\nu_{max}^{liq. film}$ : 1730, 1160, 1040 $cm^{-1}$

N.M.R. $\delta_{ppm}^{CDCl_3}$ : 1.06 to 2.06 (11H, m)
1.28 (3H, s)
2.70 (1H, d,d, J=12Hz, 14Hz)
2.96 (1H, broad s)
3.62 (3H, s)
3.64 to 3.88 (1H, d,d, J=3Hz), 12 to 14Hz)

3.90 (4H, s)
7.12 to 7.40 (3H, m)

(2) A solution of 6.597 g. methyl 2-(3-chloro-4-cyclohexylphenyl)-4,4-ethylenedioxyvalerate in 25 ml. anhydrous diethyl ether was added dropwise to a suspension of 683.1 mg. lithium aluminium hydride in 50 ml. anhydrous diethyl ether over the course of 80 minutes, with stirring and ice-cooling, whereafter the mixture was stirred at the same temperature for an hour. After decomposing excess lithium aluminium hydride by adding 4 ml. ethyl acetate, the mixture was shaken with a 15% aqueous solution of sodium hydroxide and the organic layer was separated off. The remaining aqueous layer was extracted with diethyl ether and the extract was combined with the organic layer obtained above, washed with water, dried over anhydrous magnesium sulphate and then evaporated to dryness under reduced pressure to give 5.745 g. of oily 2-(3-chloro-4-cyclohexylphenyl)-4,4-ethylenedioxypentanol.

I.R. $\nu_{max}^{liq.\ film}$ : 3400, 1120, 1040 $cm^{-1}$

N.M.R. $\delta_{ppm}^{CDCl_3}$ : 1.07 to 2.10 (10H, m)
1.26 (3H, s)
2.05 (2H, d, J=6Hz)
2.58 (1H, broad s)
2.66 to 3.18 (1H, m)
2.94 (1H, quintet, J=6Hz)
3.68 (2H, d, J=6Hz)
3.91 (4H, s)
7.08 to 7.45 (3H, m)

(3) 102 mg. Acetic anhydride were added dropwise to a solution of 216.7 mg. 2-(3-chloro-4-cyclohexylphenyl)-4,4-ethylenedioxypentanol in 2 ml. pyridine, with stirring and ice-cooling, and the mixture was stirred at the same temperature for an hour and then

at ambient temperature for 5 hours. The reaction mixture was diluted with ethyl acetate, washed successively with chilled 5% hydrochloric acid, water, an aqueous solution of sodium bicarbonate and water, dried over anhydrous magnesium sulphate and then evaporated under reduced pressure to give 197 mg. oily 1-acetoxy-2-(3-chloro-4-cyclohexylphenyl)-3-acetylpropane.

I.R. $\nu_{max}^{liq. film}$ : 1740, 1720 $cm^{-1}$

N.M.R. $\delta_{ppm}^{CDCl_3}$ : 1.08 to 2.10 (10H, m)
1.96 (3H, s)
2.05 (3H, s)
2.78 (2H, d, J=6Hz)
3.00 (1H, broad s)
3.25 to 3.69 (1H, m)
4.12 (1H, d,d, J=2Hz, 6Hz)
7.05 to 7.31 (3H, m)

Example 26.

140.5 mg. Benzoyl chloride were added to a solution of 225.4 mg. 2-(3-chloro-4-cyclohexylphenyl)-4,4-ethylene dioxypentanol in 2 ml. pyridine, with stirring and ice-cooling, and the mixture was stirred at the same temperature for an hour. Ethyl acetate was added to the reaction mixture and the mixture washed successively with cold 5% hydrochloric acid, water, an aqueous solution of sodium bicarbonate and water, dried over anhydrous magnesium sulphate and then evaporated under reduced pressure to give 232 mg. oily 1-benzoyloxy-2-(3-chloro-4-cyclohexylphenyl)-3-acetylpropane.

I. R. $_{max}^{liq. film}$ : 1720 $cm^{-1}$

N.M.R. $_{ppm}^{CDCl_3}$ : 1.07 to 2.10 (10H, m)
2.10 (3H, s)
2.87 (2H, d, J=6Hz)

2.82 to 3.46 (2H, m)
4.41 (2H, d, J=7Hz)
7.13 to 7.69 (6H, m)
7.86 to 8.17 (2H, m)

Example 27

2.741 g. Nicotinoyl chloride hydrochloride were added portionwise to a mixture of 4.739 g. 2-(3-chloro-4-cyclohexylphenyl)-4,4-ethylenedioxypentanol, 4.242 g. triethylamine and 50 ml. methylene chloride, with stirring and ice-cooling, and the mixture stirred at the same temperature for 4 hours. The reaction mixture was evaporated under reduced pressure and ethyl acetate and water were added to the resultant residue. After shaking, the organic layer was separated and the remaining aqueous layer was extracted with ethyl acetate. The extract and the organic layer were combined, washed with a saturated aqueous solution of sodium bicarbonate and then with water, dried over anhydrous magnesium sulphate and evaporated under reduced pressure. The oily residue (6.92 g.) was dissolved in 60 ml. acetone and treated with 10 ml. 10% hydrochloric acid for 1.5 hours, with stirring and ice-cooling, and the acetone then distilled off under reduced pressure. To the residue was added a mixture of ethyl acetate and a saturated aqueous solution of sodium bicarbonate, with stirring and ice-cooling, and the organic layer was separated off. The remaining aqueous layer was extracted with ethyl acetate and the extract was combined with the organic layer, washed with a saturated aqueous solution of sodium bicarbonate and then with water, dried over anhydrous magnesium sulphate and evaporated under reduced pressure. The oily residue was chromatographed on a silica gel column and eluted with chloroform to give 4.658 g. oily 1-nicotinoyloxy-2-(3-chloro-4-cyclohexylphenyl)-3-acetylpropane.

I. R. $\nu_{max}^{liq.film}$ : 1715 (broad) $cm^{-1}$

N.M.R. $\delta_{ppm}^{CDCl_3}$ : 1.06 to 2.06 (10H, m)
2.14 (3H, s)
2.78 to 3.16 (1H, m)
2.94 (2H, d, J=7Hz)
3.72 (1H, quintet, J=7Hz)
4.50 (2H, d, J=7Hz)
7.14 to 7.54 (4H, m)
8.18 to 8.36 (1H, m)
8.78 to 8.90 (1H, m)
9.20 (1H, d, J=2Hz)

Example 28

(1) A solution of 6.318 g. 1-nitropentane in 15 ml. methanol was added dropwise to a cold solution of 1.863 g. sodium in 100 ml. methanol, with stirring and ice-cooling. To the mixture was added dropwise a solution of 15.039 g. methyl 2-(3-chloro-4-cyclohexylphenyl)acrylate in 55 ml. methanol over the course of 30 minutes, with stirring and ice-cooling. After stirring for 5 hours at ambient temperature, a further solution of 1.242 g. sodium in 40 ml. methanol was added dropwise to the above mixture over the course of 30 minutes and the mixture then added dropwise to a cold mixture of 200 ml. 10% hydrochloric acid and 250 ml. methanol over the course of an hour, with stirring The resultant mixture was stirred at the same temperature for an hour and then overnight at ambient temperature. Methanol was distilled off under reduced pressure and the aqueous residue was extracted with ethyl acetate. The extract was washed successively with water, an aqueous solution of sodium bicarbonate and water, dried over anhydrous magnesium sulphate and then evaporated under reduced pressure. The oily residue was fractionated by vacuum distillation to give 8.802 g.

methyl 2-(3-chloro-4-cyclohexylphenyl)-3-pentanoylpropionate; b.p. 185 - 198°C./2 mm.Hg. The 3.10 g. of distillate at 170 - 185°C./2 mm.Hg and the 5.18 g. of residue were combined, chromatographed on a silica gel column and eluted with benzene to give a further 2.21 g. of the same compound, the total yield being 11.012 g.

I.R. $\nu_{max}^{liq.\ film}$ : 1730, 1710 $cm^{-1}$

N.M.R. $\delta_{ppm}^{CDCl_3}$ : 0.88 (3H, t, J=7Hz)
1.10 to 2.12 (14H, m)
2.42 (2H, t, J=8Hz)
2.64 (1H, d,d, J=5, 17Hz)
2.98 (1H, broad s)
3.31 (1H, d,d, J=12, 17Hz)
3.65 (3H, s)
4.06 (1H, d,d, J=5, 12Hz)
7.03 to 7.09 (3H, m)

(2) 4.01 g. Methyl 2-(3-chloro-4-cyclohexylphenyl)-3-pentanoylpropionate were added to a solution of 0.66 g. sodium hydroxide in 6 ml. water and 30 ml. methanol and the mixture heated under reflux for 30 minutes, with stirring. Methanol was distilled off under reduced pressure and the residue was dissolved in water, washed with diethyl ether, acidified with 10% hydrochloric acid and then extracted with ethyl acetate. The remaining aqueous layer was washed with ethyl acetate and the washings were combined with the ethyl acetate extract obtained above, washed with water, dried over anhydrous magnesium sulphate and then evaporated under reduced pressure. The oily residue was triturated with n-hexane and allowed to stand overnight in a refrigerator. The precipitate was filtered off, washed with n-hexane and then recrystallized from n-hexane to give 2.12 g. 2-(3-chloro-4-cyclohexylphenyl)-3-pentanoylpropionic acid; m.p. 86 - 87.5°C.

I. R. $\nu_{max}^{Nujol}$ : 1710, 1705 (sh) $cm^{-1}$

N.M.R. $\delta_{ppm}^{CDCl_3}$ : 0.86 (3H, t, J=7Hz)
1.06 to 2.09 (14H, m)
2.40 (2H, t, J=7Hz)
2.66 (1H, d,d, J=5, 17Hz)
2.97 (1H, broad s)
3.26 (1H, d,d, J=10, 17Hz)
4.06 (1H, d,d, J=5, 10Hz)
7.06 to 7.38 (3H, m)
10.25 (1H, broad s)

Example 29

2.48 g. Decanoyl chloride were added dropwise to a solution of 2.83 g. 2-(3-chloro-4-cyclohexylphenyl)-3-methoxypropanol in 15 ml. pyridine over the course of 5 minutes at 3 - 5°C., with stirring, and the mixture stirred at the same temperature for 2 hours and at ambient temperature for 15 hours. The reaction mixture was poured into a mixture of 50 ml. 10% hydrochloric acid and 50 ml. ethyl acetate, with shaking. The ethyl acetate layer was separated with ice-cooling and the remaining aqueous layer was extracted with 30 ml. ethyl acetate. The extract was combined with the ethyl acetate solution obtained above, washed 4 times with 30 ml. amounts of cold 10% hydrochloric acid, and once each with water, a saturated aqueous solution of sodium bicarbonate and water, dried over anhydrous magnesium sulphate and then evaporated under reduced pressure. The oily residue was chromatographed on a silica gel column and eluted with a mixture of n-hexane and benzene (1:1 v/v) to give 2.6 g. oily 1-decanoyloxy-2-(3-chloro-4-cyclohexylphenyl)-3-methoxypropane.

Analysis:

calc. for $C_{26}H_{41}O_3Cl$ : C 71.45%; H 9.46%; Cl 8.11%

found : C 71.30%, H 9.48%; Cl 8.08%

I.R. $\nu_{max}^{liq.film}$ : 1740 $cm^{-1}$

N.M.R. $\delta_{ppm}^{CDCl_3}$ : 0.86 (3H, t, J=5Hz)
1.07 to 2.07 (24H, m)
2.24 (2H, t, J=7Hz)
2.73 to 3.23 (2H, m)
3.30 (3H, s)
3.58 (2H, d, J=6Hz)
4.27 (2H, d, J=6Hz)
7.08 to 7.35 (3H, m)

Example 30

2.95 g. Palmitoyl chloride were added dropwise to a solution of 2.5 g. 2-(3-chloro-4-cyclohexylphenyl)-3-methoxypropanol in 13.5 ml. pyridine at 3 - 5°C. over the course of 5 minutes and the mixture stirred at the same temperature for 6 hours. The reaction mixture was treated in the same manner as described in Example 29 to give 5.2 g. of an oily residue which was chromatographed on a silica gel column and eluted with a mixture of benzene and *n*-hexane to give 3.5 g. oily 1-palmitoyloxy-2-(3-chloro-4-cyclohexylphenyl)-3-methoxypropane.

Analysis:

calc. for $C_{32}H_{53}O_3Cl$ : C 73.74%; H 10.23%; Cl 6.80%
found : 73.69%; 10.45%; 7.05%

I.R. $\nu_{max}^{liq.film}$ : 1750 $cm^{-1}$

N.M.R. $\delta_{ppm}^{CDCl_3}$ : 0.88 (3H, t, J=5Hz)
1.00 to 2.01 (36H, m)
2.26 (2H, t, J=7Hz)
2.80 to 3.24 (2H, m)
3.34 (3H, s)

3.61 (2H, d, J=6Hz)
4.32 (2H, d, J=7Hz)
7.11 to 7.30 (3H, m)

Example 31

A solution of 2.566 g. propyl 2-(3-chloro-4-cyclohexylphenyl)-3-propoxypropionate in 15 ml. anhydrous diethyl ether was added dropwise over the course of 20 minutes to a cold suspension of 265.7 mg. lithium aluminium hydride in 20 ml. anhydrous diethyl ether, with stirring and ice-cooling, and the mixture stirred at the same temperature for 30 minutes. After decomposing excess lithium aluminium hydride with ethyl acetate, the reaction mixture was shaken with 10% hydrochloric acid and the organic layer was separated. The remaining aqueous layer was extracted with diethyl ether and the extract was combined with the organic layer obtained above, washed with water, dried over anhydrous magnesium sulphate and then evaporated to dryness under reduced pressure to give 2.13 g. oily 2-(3-chloro-4-cyclohexylphenyl)-3-propoxypropanol.

I.R. $\nu_{max}^{liq.film}$ : 3400 $cm^{-1}$

N.M.R. $\delta_{ppm}^{CDCl_3}$ : 0.91 (3H, t, J=7Hz)
1.13 to 2.13 (12H, m)
2.67 (1H, t, J=6Hz)
2.73 to 3.30 (2H, m)
3.42 (2H, t, J=6Hz)
3.63 to 4.08 (4H, m)
7.08 to 7.43 (3H, m)

Example 32

To a cold solution of 1.208 g. sodium in 100 ml. n-propanol was added dropwise a solution of 5.849 g. methyl 2-(3-chloro-4-cyclohexylphenyl) acrylate in 20 ml. n-propanol, with stirring and ice-cooling, and

the mixture was stirred at ambient temperature for 4 hours. After neutralizing with 3.3 ml. acetic acid, the mixture was evaporated under reduced pressure. The residue was shaken with a mixture of ethyl acetate and water and the ethyl acetate layer was separated. The remaining aqueous layer was extracted with ethyl acetate and the extract was combined with the ethyl acetate layer obtained above, washed with an aqueous sodium bicarbonate solution and water, dried over anhydrous magnesium sulphate and then evaporated under reduced pressure. The oily residue was chromatographed on a silica gel column and eluted with a mixture of n-hexane and benzene (2.5:1 v/v) to give 3.266 g. oily propyl 2-(3-chloro-4-cyclohexylphenyl)-3-propoxypropionate.

I.R. $\nu_{mad}^{liq.\ film}$ : 1740 $cm^{-1}$

N.M.R. $\delta_{ppm}^{CDCl_3}$: 0.87 (6H, t, J=7Hz)
1.12 to 2.18 (14H, m)
2.76 to 3.20 (1H, m)
3.40 (2H, t, J=7Hz)
3.54 to 4.31 (5H, m)
7.17 to 7.50 (3H, m)

Example 33

(1) A solution of 1.69 g. diethyl malonate in 2 ml. N,N-dimethylformamide was added dropwise over the course of 5 minutes to a suspension of 506.9 mg. 50% sodium hydride in 10 ml. N,N-dimethylformamide, with stirring and ice-cooling. To the mixture was added dropwise a solution of 3.841 g. 1-tosyloxy-2-(3-chloro-4-cyclohexylphenyl)-3-methoxypropane in 15 ml. N,N-dimethylformamide over the course of 15 minutes, with stirring and ice-cooling, and the mixture stirred at ambient temperature for 10 minutes and then for 3 hours in an oil bath at 100°C. The reaction mixture was shaken with

a mixture of water and ethyl acetate and the organic layer was separated. The remaining aqueous layer was extracted with ethyl acetate and the extract was combined with the organic layer obtained above, washed successively with water, a saturated aqueous solution of sodium bicarbonate and water, dried over anhydrous magnesium sulphate and then evaporated under reduced pressure. Excess diethyl malonate was then also distilled off under reduced pressure. The oily residue obtained was dissolved in a small amount of benzene, chromatographed on a silica gel column and then eluted with benzene to give 2.27 g. oily diethyl 3-(3-chloro-4-cyclohexylphenyl)-4-methoxybutane-1,1-dicarboxylate.

I. R. $\nu_{max}^{liq.film}$ : 1745, 1720 $cm^{-1}$

N.M R. $\delta_{ppm}^{CDCl_3}$ : 1.18 (3H, t, J=7Hz)
1.25 (3H, t, J=7Hz)
0.97 to 2.08 (10H, m)
2.11 to 2.46 (2H, m)
2.61 to 3.59 (5H, m)
3.28 (3H, s)
4.07 (2H, q, J=7Hz)
4.20 (2H, q, J=7Hz)
7.02 to 7.45 (3H, m)

(2) A mixtu-e of a solution of 5.094 g. diethyl 3-(3-chloro-4-cyclohexylphenyl)-4-methoxybutane-1,1-dicarboxylate in 50 ml. methanol and a solution of 1.44 g. sodium hydroxide in 10 ml. water was heated under reflux for an hour, with stirring, and then evaporated under reduced pressure. The residue was acidified with 10% hydrochloric acid, with ice-cooling, and then extracted with ethyl acetate. The extract was washed with water, dried over anhydrous magnesium sulphate and then evaporated under reduced pressure. The oily residue was triturated with a small amount of

n-hexane, while cooling, and the precipitate was filtered off and washed with n-hexane to give 4.073 g. 3-(3-chloro-4-cyclohexylphenyl)-4-methoxybutane-1,1-dicarboxylic acid; m.p. 85 - 88°C.

I.R. $\nu_{max}^{Nujol}$ : 1720, 1745 $cm^{-1}$

N.M.R. $\delta_{ppm}^{DMSO-d_6}$: 1.10 to 2.31 (12H, m)
2.63 to 3.13 (3H, m)
3.18 (3H, s)
3.45 (2H, d, J=6Hz)
7.12 to 7.53 (3H, m)

(3) A solution of 3.685 g. 3-(3-chloro-4-cyclohexylphenyl)-4-methoxybutane-1,1-dicarboxylic acid in 10 ml. xylene was heated under reflux for 30 minutes and then evaporated under reduced pressure. The oily residue was dissolved in 10 ml. methanol and mixed with a solution of 380 mg. sodium hydroxide in 10 ml. methanol. The mixture was stirred for some time and then evaporated to dryness under reduced pressure. The residue was triturated with n-hexane with cooling, and the precipitate was filtered off, recrystallized from a mixture of diethyl ether and n-hexane and then washed with n-hexane to give 2.779 g. sodium 4-(3-chloro-4-cyclohexylphenyl)-5-methoxyvalerate; m.p. 273 - 276°C. (dec.).

I.R. $\nu_{max}^{Nujol}$ : 1590 $cm^{-1}$

N.M.R. $\delta_{ppm}^{D_2O}$ : 0.89 to 2.36 (12H, m)
2.53 to 3.59 (6H, m)
3.13 (3H, s)
6.86 to 7.30 (3H, m)

Example 34

To a cold solution of 3.45 g. sodium in 100 ml. anhydrous n-propanol was added dropwise over the course of 20 minutes a solution of 12.75 g. methyl 2-(4-cyclohexylphenyl)acrylate in 30 ml. n-propanol with stirring and ice-cooling, and the mixture was stirred at ambient temperature for 7 hours. The reaction mixture was treated in a similar manner to that of Example 32 to give 11.56 g. oily n-propyl 2-(4-cyclohexylphenyl)-3-n-propoxypropionate.

I.R. $\nu_{max}^{liq.\ film}$ : 1730 $cm^{-1}$

N.M.R. $\delta_{ppm}^{CDCl_3}$ : 0.83 (6H, t, J=8Hz)
1.04 to 2.10 (14H, m)
2.27 to 2.68 (1H, m)
3.38 (2H, t, J=8Hz)
3.51 to 4.23 (5H, m)
7.04 to 7.35 (4H, m)

Example 35

A solution of 4.761 g. sodium in 100 ml. anhydrous methanol was added dropwise over the course of 20 minutes to a solution of 23.357 g. methyl 2-(3-chloro-4-cyclohexylphenyl)-3-acetoxypropionate in 25 ml. anhydrous methanol with stirring and ice-cooling. The mixture was treated in a similar manner to that of Example 17-(1) to give 20.99 g. oily residue. The residue was dissolved in a small amount of n-hexane, the solution was chromatographed on a silica gel column and then eluted successively with n-hexane, n-hexane and benzene (1:1 v/v) and n-hexane and benzene (1:2 v/v). The fraction of n-hexane and benzene (1:2 v/v) was evaporated to dryness under reduced pressure to give 12.98 g. oily methyl 2-(3-chloro-4-cyclohexylphenyl)-3-methoxypropionate.

I.R. $\nu_{max}^{liq.\ film}$ : 1735 $cm^{-1}$

N.M.R. $\delta_{ppm}^{CDC\ell_3}$ : 1.04 to 2.10 (10H, m)
2.96 (1H, broad s)
3.34 (3H, s)
3.48 to 4.04 (3H, m)
3.68 (3H, s)
7.06 to 7.46 (3H, m)

Example 36

A solution of 4.954 g. decanoyl chloride in 5 ml. methylene chloride was added dropwise over the course of 1 hour to a solution of 3.222 g. 2-(3-chloro-4-cyclohexylphenyl)propane-1,3-diol and 2.666 g. triethylamine in 25 ml. methylene chloride, with stirring and ice-cooling, stirred at the same temperature for 4 hours. The solvent was distilled off under reduced pressure, and ethyl acetate and water were added to the resultant residue. After shaking, the organic layer was separated and washed successively with an aqueous solution of sodium bicarbonate, water, 10% hydrochloric acid, and water, dried over anhydrous magnesium sulphate and evaporated. The oily residue was dissolved in a small amount of n-hexane, the solution was chromatographed on a silica gel column and eluted successively with n-hexane, n-hexane and benzene (2:1 v/v), n-hexane and benzene (1:1 v/v) to give 4.387 g. oily 1,3-di-n-decanoyloxy-2-(3-chloro-4-cyclohexylphenyl)propane.

Analysis:

Calc. for $C_{35}H_{57}C\ell O_4$:
C 72.82%; H 9.95%; Cℓ 6.14%

found : C 72.61%; H 9.90%, Cℓ 6.70%

I.R. $\nu_{max}^{liq.\ film}$ : 1740 $cm^{-1}$

N.M.R. $\delta_{ppm}^{CDC\ell_3}$ : 0.89 (6H, t, J=6Hz)
1.07 to 2.10 (38H, m)
2.30 (4H, t, J=7Hz)

2.79-3.53 (2H, m)
4.35 (4H, d, J=7Hz)
7.13-7.38 (3H, m)

Example 37

1.632 g. 50% sodium hydride was added to a solution of 10.74 g. 2-(3-chloro-4-cyclohexylphenyl)-propane-1,3-diol in 50 ml. dimethylformamide and stirred for 10 minutes under ice-cooling. 10 ml. Petroleum ether was added to the mixture and stirred at ambient temperature for 1.5 hours. 5.436 g. n-Pentyl bromide was added dropwise to the mixture over the course of 30 minutes, and stirred at ambient temperature for 3 hours. Water and ethyl acetate were added to the reaction mixture. After shaking, the organic layer was separated, washed with water, dried over anhydrous magnesium sulphate and then evaporated to dryness under reduced pressure. The oily residue was dissolved in n-hexane and the solution was left to stand at ambient temperature, filtered and then washed with n-hexane. The filtrate and the washings were combined and evaporated under reduced pressure. The residue was dissolved in a small amount of benzene, chromatographed on a silica gel column and eluted successively with benzene and chloroform to give 5.61 g. 3-n-pentyloxy-2-(3-chloro-4-cyclohexylphenyl)propanol.

Analysis:

Calc. for $C_{20}H_{31}C\ell O_2$ : C 70.88%; H 9.22 %; Cℓ 10.46%

found : C 70.93%; H 9.28%; Cℓ 10.64%

I.R. $\nu_{max}^{liq.\ film}$ : 3400 $cm^{-1}$

N.M.R. $\delta_{ppm}^{CDC\ell_3}$ : 0.89 (3H, t, J=7Hz)
1.03-2.08 (16H, m)
2.71 (1H, t, J=6Hz)
2.80-3.24 (2H, m)
3.43 (2H, t, J=6Hz)

3.58-4.06 (4H, m)
6.98-7.30 (3H, m)

Example 38

(1) A solution of 2.133 g. benzoylchloride in 10 ml. methylene chloride was added dropwise over the course of 20 minutes to a mixture of 4.078 g. methyl 2-(3-chloro-4-cyclohexylphenyl)-3-aminopropionate, 1.673 g. triethylamine and 30 ml. methylene chloride, with stirring and ice-cooling, stirred at the same temperature for 30 minutes and further at ambient temperature for 1 hour, and then left to stand overnight at the same temperature. The reaction mixture was treated in a similar manner to that of Example 14-(2) to give 6.03 g. oily methyl 2-(3-chloro-4-cyclohexylphenyl)-3-benzamidopropionate.

I.R. $\nu_{max}^{liq. film}$ : 3300, 1725 $cm^{-1}$

(2) A solution of 5.993 g. methyl 2-(3-chloro-4-cyclohexylphenyl)-3-benzamidopropionate and 1.2 g. sodium hydroxide in 30 ml. methanol and 10 ml. water was heated under reflux for 30 minutes, while stirring. Methanol was distilled off under reduced pressure. The residual mixture was adjusted to pH 1 with 10% hydrochloric acid and extracted with ethyl acetate. The extract was washed with water, dried over anhydrous magnesium sulfate and then evaporated under reduced pressure. Precipitated crystals were recrystallized from methanol to give 2.59 g. 2-(3-chloro-4-cyclohexylphenyl)-3-benzamidopropionic acid; mp. 197.5-199°C.

Analysis:

Calc. for $C_{22}H_{24}C\ell NO_3$ : C 68.48%; H 6.27%; N 3.63%; Cℓ 9.19%

found : C 68.32%; H 6.12%; N 3.64%; Cℓ 9.36%

I.R. $\nu_{max}^{Nujol}$ : 3360, 1700, 1640 $cm^{-1}$

N.M.R. $\delta_{ppm}^{DMSO}$ : 0.98 to 1.98 (10H, m)
2.88 (1H, broad, s)
3.26 to 3.84 (2H, m)
3.94 (1H, d,d, J=7Hz, 15Hz)
7.10 to 7.54 (6H, m)
7.60 to 7.86 (2H, m)
8.34 to 8.66 (1H, m)

Example 39

(1) A solution of 1.14 g. methyl isocyanate in 10 ml. methylene chloride was added dropwise over the course of 15 minutes to a solution of 5.91 g. methyl 2-(3-chloro-4-cyclohexylphenyl)-3-aminopropionate in 30 ml. methylene chloride, with stirring and ice-cooling, and stirred at the same temperature for 25 minutes. The reaction mixture was evaporated to dryness under reduced pressure to give 6.86 g. oily methyl 2-(3-chloro-4-cyclohexylphenyl)-3-(3-methylureido)propionate.

I.R. $\nu_{max}^{CHCl_3}$ : 3350, 1720, 1630 $cm^{-1}$

N.M.R. $\delta_{ppm}^{DMSO}$ : 1.06 to 2.08 (10H, m)
2.53 (3H, d, J=4Hz)
2.88 (1H, broad s)
3.24 to 3.68 (2H, m)
3.61 (3H, s)
3.84 (1H, t, J=9Hz)
5.78 (1H, q, J=4Hz)
6.02 (1H, t, J=6Hz)
7.12 to 7.40 (3H, m)

(2) A solution of 6.698 g. methyl 2-(3-chloro-4-cyclohexylphenyl)-3-(3-methylureido)propionate and 1.14 g. sodium hydroxide in 50 ml. methanol was heated under reflux for 1 hour, while stirring. Methanol was distilled off under reduced pressure, and the residue was dissolved in water, washed with ethyl acetate and then adjusted to pH 1 with 10% hydrochloric acid. The precipitated crystals were collected by filtration with

suction, washed with water, dried, and recrystallized from methanol to give 3.220 g. 2-(3-chloro-4-cyclohexylphenyl)-3-(3-methylureido)propionic acid; mp. 188.5 - 190°C (decomp.).

Analysis:

Calc. for $C_{17}H_{23}ClN_2O_3$ : C 60.26%; H 6.84%; N 8.27%; Cl 10.46%

found : C 60.37%; H 6.96%; N 8.18%; Cl 10.57%

I.R. $\nu_{max}^{Nujol}$ : 3400, 1735, 1580 $cm^{-1}$

N.M.R. $\delta_{ppm}^{DMSO}$: 0.96 to 2.00 (10H, m)
2.51 (3H, d, J=5Hz)
2.90 (1H, broad s)
3.18 to 3.58 (2H, m)
3.70 (1H, d,d, J=7, 12Hz)
5.84 (1H, q, J=5Hz)
6.00 (1H, t, J=5Hz)
7.28 to 7.46 (3H, m)

Example 40

A mixture of 3.855 g. 2-(3-chloro-4-cyclohexylphenyl)-3-methoxypropionic acid, 20 ml. n-butanol and 1 ml. conc. sulfuric acid was stirred at 100°C for 30 minutes. The reaction mixture was evaporated under reduced pressure and the residue was shaken with a mixture of ethyl acetate and cold water. The organic layer was separated,washed successively with water, a saturated aqueous solution of sodium bicarbonate and water, dried over anhydrous magnesium sulphate and then evaporated under reduced pressure. The oily residue was fractionated by vacuum distillation to give 2.833 g. n-butyl 2-(3-chloro-4-cyclohexylphenyl)-3-methoxypropionate; b.p. 185°-187°C/5 mm.Hg.

I.R. $\nu_{max}^{Nujol}$ : 1745 $cm^{-1}$

N.M.R. $\delta_{ppm}^{CDCl_3}$ : 0.87 (3H, t, J=6Hz)

1.10 to 2.07 (1H, m)
2.73 to 3.10 (1H, m)
3.36 (3H, s)
3.50 to Ca. 4.00 (3H, m)
4.13 (2H, t, J=6Hz)
7.15 to 7.41 (3H, m)

## CLAIMS

1. A compound of the formula:-

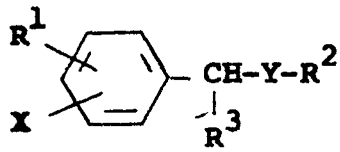

wherein $R^1$ is a lower cycloalkyl radical,
$R^2$ is a carboxy, esterified carboxy, hydroxymethyl, lower alkoxymethyl or acyloxymethyl radical,
$R^3$ is a lower alkanoyl radical or a lower alkyl radical substituted with a hydroxy, amino, lower alkylamino, di(lower)-alkylamino, acylamino, lower alkoxy, acyl or acyloxy radical, in which the lower alkyl moieties of the di(lower)alkyl amino radical may be joined together to form a heterocyclic ring containing the nitrogen atom, and the carbonyl function of the acyl radical, which is a substituent on the lower alkyl radical, may be protected,
X is a hydrogen or halogen atom, and
Y is a valency bond or a lower alkylene radical;

and the pharmaceutically acceptable salts thereof.

2. A process for preparing a compound of the formula:-

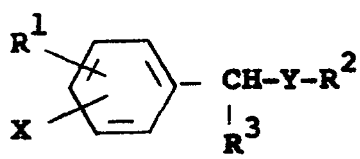

wherein $R^1$ is a lower cycloalkyl radical,
$R^2$ is a carboxy, esterified carboxy, hydroxymethyl, lower alkoxymethyl or acyloxymethyl radical,

$R^3$ is a lower alkanoyl radical or a lower alkyl radical substituted with a hydroxy, amino, lower alkylamino, di(lower)alkylamino, acylamino, lower alkoxy, acyl or acyloxy radical, in which the lower alkyl moieties of the di(lower)alkylamino radical may be joined together to form a heterocyclic ring containing the nitrogen atom, and the carbonyl function of the acyl radical, which is a substituent on the lower alkyl radical may be protected,

X is a hydrogen or halogen atom, and

Y is a valency bond or a lower alkylene radical;

or a pharmaceutically acceptable salt thereof, which comprises

(1) reacting a compound of the formula:-

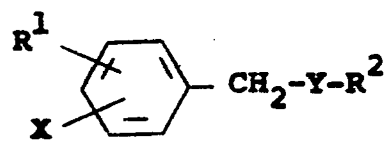

wherein $R^1$, $R^2$, X and Y have the same meanings as above, with a lower alkanoic acid of the formula:-

$$R^3_a - OH$$

wherein $R^3_a$ is a lower alkanoyl radical, or a reactive derivative thereof to give a compound of the formula:-

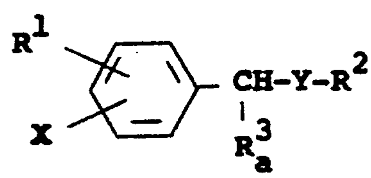

wherein $R^1$, $R^2$, $R^3_a$, X and Y have the same meanings as above;

(2) reacting a compound of the formula:-

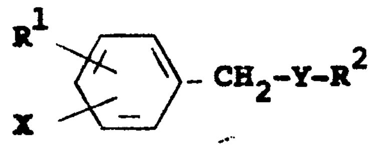

wherein $R^1$, $R^2$, X and Y have the same meanings as above, with paraformaldehyde to give a compound of the formula:-

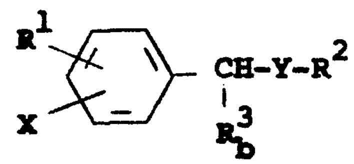

wherein $R^1$, $R^2$, $R_b^3$, X and Y have the same meanings as above;

(3) reducing a compound of the formula:-

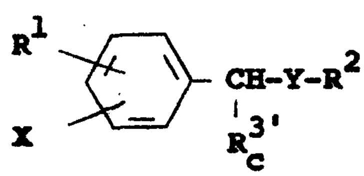

wherein $R^1$, $R^2$, X and Y have the same meanings as above and $R_c^{3'}$ is a lower alkanoyl radical, to give a compound of the formula:-

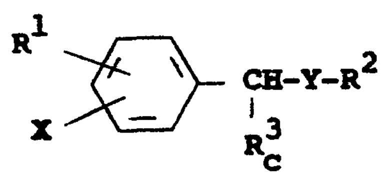

wherein $R^1$, $R^2$, X and Y have the same meanings as above and $R_c^3$ is a hydroxy(lower)alkyl radical, or a compound of the formula:-

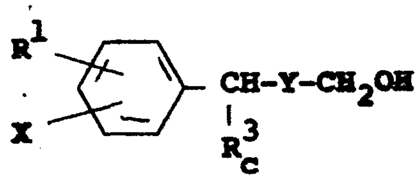

wherein $R^1$, $R^3_c$, X and Y have the same meanings as above;

(4) reacting a compound of the formula:-

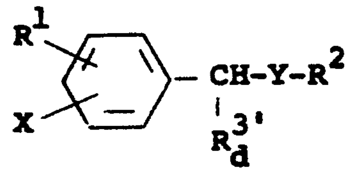

wherein $R^1$, $R^2$, X and Y have the same meanings as above and $R^{3'}_d$ is a hydroxy(lower)alkyl or acyloxy-(lower)alkyl radical, with an alkylating agent to give a compound of the formula:-

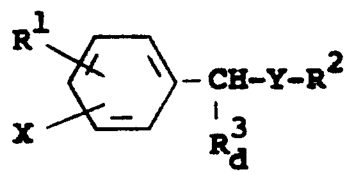

wherein $R^1$, $R^2$, X and Y have the same meanings as above and $R^3_d$ is a lower alkoxy(lower)alkyl radical;

(5) reacting a compound of the formula:-

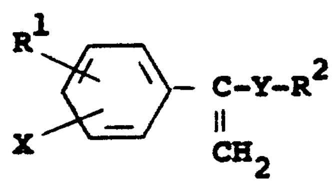

wherein $R^1$, $R^2$, X and Y have the same meanings as above, with an alkylating agent to give a compound of the formula:-

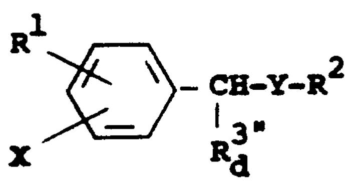

wherein $R^1$, $R^2$, X and Y have the same meanings as above and $R^{3''}_d$ is a lower alkoxymethyl radical;

(6) reacting a compound of the formula:-

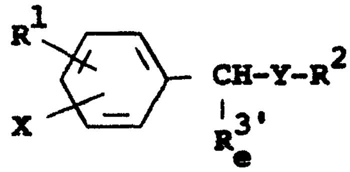

wherein $R^1$, $R^2$, X and Y have the same meanings as above and $R_e^{3'}$ is a hydroxy(lower)alkyl radical, with an acylating agent to give a compound of the formula:-

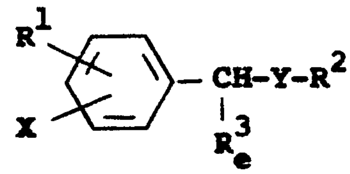

wherein $R^1$, $R^2$, X and Y have the same meanings as above and $R_e^3$ is an acyloxy(lower)alkyl radical;

(7) reacting a compound of the formula:-

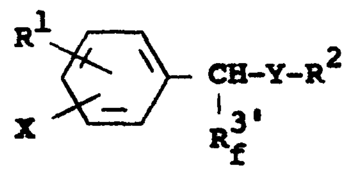

wherein $R^1$, $R^2$, X and Y have the same meanings as above and $R_f^{3'}$ is an acyloxy(lower)alkyl radical, with ammonia or an amine compound selected from alkylamines, di(lower)alkylamines and saturated heterocyclic imine compounds containing an imino group, or a compound of the formula:-

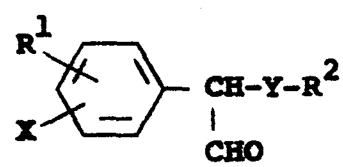

wherein $R^1$, $R^2$, X and Y have the same meanings as above, with ammonia or a lower alkylamine, di(lower)-alkylamine or saturated heterocyclic compound containing an imino group, in the presence of a reducing

agent to give a compound of the formula:-

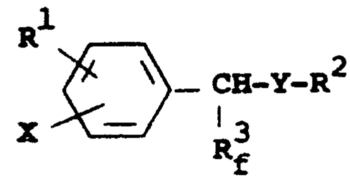

wherein $R^1$, $R^2$, X and Y have the same meanings as above and $R_f^3$ is a lower alkyl radical substituted by an amino, lower alkylamino or di(lower)alkylamino group, in which the lower alkyl moieties of the di(lower)alkylamino group can be joined together to form a heterocyclic ring containing the nitrogen atom;

(8) reacting a compound of the formula:-

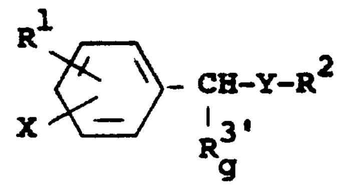

wherein $R^1$, $R^2$, X and Y have the same meanings as above and $R_g^{3'}$ is an amino(lower)alkyl radical, with an acylating agent to give a compound of the formula:-

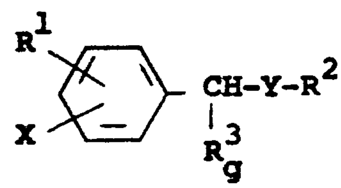

wherein $R^1$, $R^2$, X and Y have the same meanings as above and $R_g^3$ is an acylamino(lower)alkyl radical;

(9) reacting a compound of the formula:-

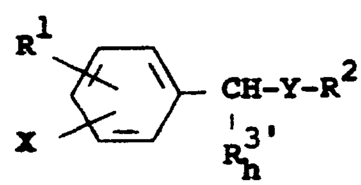

wherein $R^1$, $R^2$, X and Y have the same meanings as above and $R_h^{3'}$ is a hydroxy(lower)alkyl or acyloxy-(lower)alkyl radical, or a compound of the formula:-

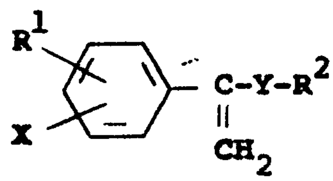

wherein $R^1$, $R^2$, X and Y have the same meanings as above, with an aryl-substituted or unsubstituted nitro(lower)alkane in the presence of a strong base, followed by treating the resultant compound with a mineral acid or an oxidising agent to give a compound of the formula:-

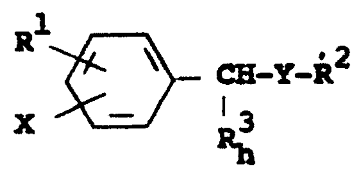

wherein $R^1$, $R^2$, X and Y have the same meanings as above and $R_h^3$ is an acyl(lower)alkyl radical;

(10) hydrolysing a compound of the formula:-

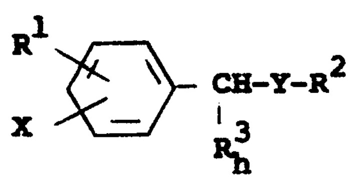

wherein $R^1$, $R^3$, X and Y have the same meanings as above and $R_i^{2'}$ is an esterified carboxy group, to give a compound of the formula:-

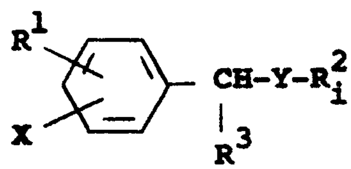

wherein $R^1$, $R^3$, X and Y have the same meanings as above and $R_i^2$ is a carboxy radical;

(11) reacting a compound of the formula:-

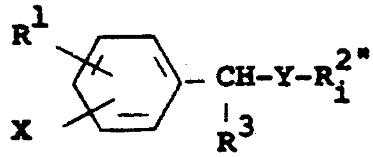

wherein $R^1$, $R^3$, X and Y have the same meanings as above and $R_i^{2"}$ is an acyloxymethyl radical, with a diester of malonic acid in the presence of a base, and then hydrolysing the resultant compound of the formula:-

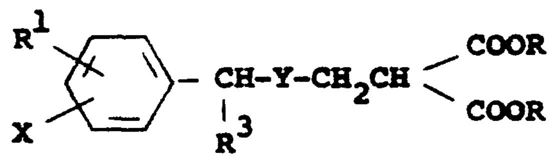

wherein $R^1$, $R^3$, X and Y have the same meanings as above and COOR is an esterified carboxy group, and finally decarboxylating the resultant dicarboxylic acid to give a compound of the formula:-

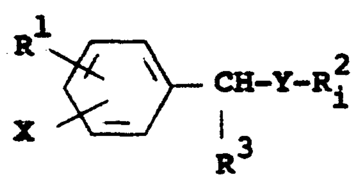

wherein $R^1$, $R_i^2$, $R^3$, X and Y have the same meanings as above;

(12) reducing a compound of the formula:-

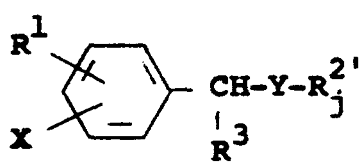

wherein $R^1$, $R^3$, X and Y have the same meanings as above and $R_j^{2'}$ is a carboxy or esterified carboxy group to give a compound of the formula:-

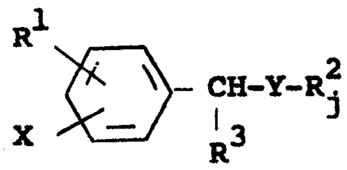

wherein $R^1$, $R^3$, X and Y have the same meanings as above and $R^2_j$ is a hydroxymethyl radical;

(13) acylating a compound of the formula:-

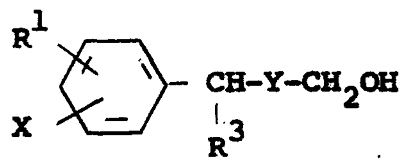

wherein $R^1$, $R^3$, X and Y have the same meanings as above, to give a compound of the formula:-

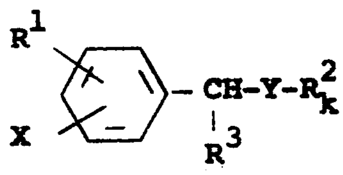

wherein $R^1$, $R^3$, X and Y have the same meanings as above and $R^2_k$ is an acyloxymethyl radical;

(14) oxidising a compound of the formula:-

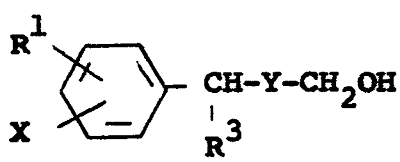

wherein $R^1$, $R^3$, X and Y have the same meanings as above, to give a compound of the formula:-

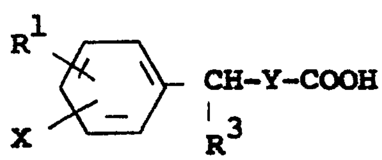

wherein $R^1$, $R^3$, X and Y have the same meanings as above, or

(15) esterifying a compound of the formula:-

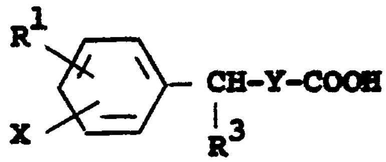

wherein $R^1$, $R^3$, X and Y have the same meanings as above, to give a compound of the formula:-

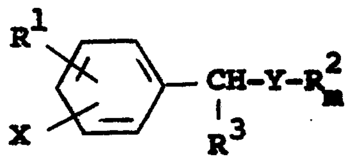

wherein $R^1$, $R^3$, X and Y have the same meanings as above, and $R^2_m$ is an esterified carboxy radical.

3. A pharmaceutical composition comprising a compound of the claim 1 as active ingredient, in association with a pharmaceutically-acceptable, substantially non-toxic carrier or excipient.

4. A method for treating an inflammation, which comprises administering a compound of the claim 1 to a mammal.

5. Sodium 2-(3-chloro-4-cyclohexylphenyl)-3-methoxypropionate.

6. 2-(3-Chloro-4-cyclohexylphenyl)-3-methoxypropionic acid.

7. Methyl 2-(3-chloro-4-cyclohexylphenyl)-3-methoxypropionate.

8. n-Butyl 2-(3-chloro-4-cyclohexylphenyl)-3-methoxypropionate.